(19) Europäisches Patentamt
European Patent Office
Office européen des brevets

(11) Veröffentlichungsnummer: **0 001 991**
**B1**

(12) **EUROPÄISCHE PATENTSCHRIFT**

(45) Veröffentlichungstag der Patentschrift: **13.10.82**

(21) Anmeldenummer: **78101232.3**

(22) Anmeldetag: **26.10.78**

(51) Int. Cl.³: **C 07 F 9/54,** C 07 F 9/65, C 07 C 143/36, C 07 D 249/06, C 07 D 405/04, C 07 D 317/56, C 07 D 319/08

(54) **Phosphoniumverbindungen, Verfahren zu deren Herstellung und Verfahren zur Herstellung von unsymmetrisch substituierten Stilbenaufhellern.**

(30) Priorität: **04.11.77 LU 78449**

(43) Veröffentlichungstag der Anmeldung:
**30.05.79 Patentblatt 79/11**

(45) Bekanntmachung des Hinweises auf die Patenterteilung:
**13.10.82 Patentblatt 82/41**

(84) Benannte Vertragsstaaten:
**CH DE FR GB**

(56) Entgegenhaltungen:
**DE - A - 2 261 062**
**FR - A - 2 258 392**
**US - A - 3 843 633**
**US - A - 3 843 718**
**US - A - 3 984 399**

(73) Patentinhaber: **CIBA-GEIGY AG**
**Patentabteilung Postfach**
**CH-4002 Basel (CH)**

(72) Erfinder: **Märky, Michael, Dr.**
**Bollwerkstrasse 50/2**
**CH-4102 Binningen (CH)**

**EP 0 001 991 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Phosphoniumverbindungen, Verfahren zu deren Herstellung und Verfahren zur Herstellung von unsymmetrisch substituierten Stilbenaufhellern

Die Erfindung betrifft neue Phosphoniumverbindungen, ein Verfahren zu deren Herstellung sowie ein Verfahren zur Herstellung von unsymmetrisch substituierten Stilbenaufhellern.

Die neuen Phosphoniumverbindungen entsprechen der Formel

$$(R_3)\overset{\oplus}{P}—CH_2—X—CH{=}CH—R_1—Y^{\ominus} \qquad (1)$$

worin R Alkyl mit 1 bis 6 C-Atomen, Cycloalkyl mit 5 bis 7 C-Atomen, $—NX_1X_2$, worin $X_1$ und $X_2$ unabhängig voneinander für Alkyl mit 1 bis 4 C-Atomen oder zusammen mit dem N-Atom für einen 5- oder 6-gliedrigen gesättigten heterocyclischen Ring stehen; oder unsubstituiertes oder durch Alkyl mit 1—6 C-Atomen, Alkoxy mit 1—6 C-Atomen, Chlor, Fluor, Brom, Jod, Cycloalkyl mit 5—7 Ringgliedern, Phenyl, Phenoxy, Naphthoxy oder durch die Gruppe $—NX_1X_2$, worin $X_1$ und $X_2$ wie vorstehend definiert sind, substituiertes Phenyl,

$R_1$ einen gegebenenfalls neben der Gruppe $Y^{\ominus}$ weitere nicht-chromophore Substituenten aufweisenden Phenyl-, Naphthyl-, Furan-, Thiophen-, Oxazol-, Isoxazol-, Pyrazol-, Imidazol-, Triazol- oder Oxadiazolrest, wobei zwei Substituenten in ortho-Stellung auch die Ergänzung zu einem unsubstituierten oder nicht-chromophor substituierten Benzo- oder Naphthorest bilden können.

X Phenylen, 4,4'-Biphenylen oder 1,4- oder 2,6-Naphthylen und $Y^{\ominus}$ $SO_3^{\ominus}$, $SO_2^{\ominus}$ oder $COO^{\ominus}$ bedeuten.

Als Alkylreste R sind solche mit 1 bis 4 C-Atomen bevorzugt, insbesondere der Isopropyl- und n-Butylrest.

Als bevorzugter Cycloalkylrest ist der Cyclohexylrest zu nennen.

Als 5- oder 6-gliedrige gesättigte heterocyclische Ringe $—NX_1X_2$ kommen vorzugsweise der Pyrrolidin-, Morpholin- und Piperidinrest in Betracht.

Die Verbindungen der Formel (1) liegen als innere Salze vor.

Als nicht-chromophore Substituenten der Reste $R_1$ seien, neben dem obligatorischen Substituenten $Y^{\ominus}$, beispielsweise die folgenden erwähnt: Chlor, Brom, Fluor, $C_1$—$C_8$ Alkyl, $C_3$—$C_7$-Alkenyl, $C_5$—$C_7$ Cycloalkyl, $C_1$—$C_8$ Alkoxy, $C_2$—$C_{12}$ Alkoxyalkoxy, Phenoxy, Phenyl, Cyano, Carboxy, Carbalkoxy, Sulfo. Bevorzugte Substituenten sind im allgemeinen $C_1$—$C_4$ Alkyl oder Alkoxy, $C_3$ oder $C_4$ Alkenyl, Fluor, Chlor, Brom, $C_2$—$C_5$-Carbalkoxy und Sulfo. Falls $R_1$ neben $Y^{\ominus}$ noch Substituenten trägt, sind dies vorzugsweise 1 oder 2 Substituenten.

Unter "Sulfo" und "Carboxy" sind die Gruppen $—SO_3H$ bzw. $—COOH$ sowie deren Salze, insbesondere deren Alkalimetall-, Erdalkalimetall-, Ammonium- oder Aminsalze zu verstehen.

Als Gruppe $Y^{\ominus}$ sind die Gruppe $SO_3^{\ominus}$ und $COO^{\ominus}$, insbesondere die Gruppe $SO_3^{\ominus}$ bevorzugt.

Im Rahmen der Verbindungen der Formel (1) sind solche der Formel

$$(R')_3\overset{\oplus}{P}—CH_2—X''—CH{=}CH—R_1'—Y^{\ominus} \qquad (2)$$

worin $R_1'$ einen gegebenenfalls neben der Gruppe $Y^{\ominus}$ einen oder zwei weitere nicht-chromophore Substituenten tragenden Phenyl-, Naphthyl-, Furan-, Thiophen-, Oxazol-, Isoxazol-, Pyrazol-, Imidazol-, Triazol- oder Oxadiazolrest, wobei zwei benachbarte Substituenten auch die Ergänzung zu einem ankondensierten Benzo- oder Naphthorest bilden können.

R' Alkyl mit 1 bis 4 C-Atomen, Cyclohexyl oder Phenyl,

$Y^{\ominus}$ $SO_3^{\ominus}$, $SO_2^{\ominus}$ oder $COO^{\ominus}$ und

X'' 1,4-Phenylen, 2,6-Naphthylen oder 4,4'-Biphenylen bedeuten, sowie solche der Formel

$$(R')_3\overset{\oplus}{P}—CH_2—X'—CH{=}CH—R_1''—Y_1^{\ominus} \qquad (3)$$

worin $R_1''$ einen gegebenenfalls neben der Gruppe $Y_1^{\ominus}$ einen oder zwei weitere Substituenten aus der Gruppe Alkyl mit 1 bis 4 C-Atomen, Alkoxy mit 1 bis 4 C-Atomen, Cyclohexyl, Alkenyl mit 3 oder 4 C-Atomen, Phenyl, Sulfo, Fluor, Chlor, Brom oder Carbalkoxy mit 2 bis 5 C-Atomen tragenden Phenyl- oder Furanrest, R' Alkyl mit 1 bis 4 C-Atomen, Cyclohexyl oder Phenyl,

$Y_1^{\ominus}$ $SO_3^{\ominus}$ oder $COO^{\ominus}$ und

X' 1,4-Phenylen oder 4,4'-Biphenylen bedeuten, hervorzuheben.

Bevorzugt sind hierbei Verbindungen der Formel

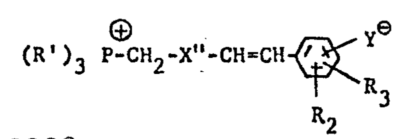

(5)

worin $Y^{\ominus}$ $—SO_3^{\ominus}$ $—SO_2^{\ominus}$ oder $COO^{\ominus}$,

$R_2$ Wasserstoff, Chlor, Brom, Fluor, Alkyl oder Alkoxy mit jeweils 1 bis 4 C-Atomen oder eine weitere Gruppe $Y^{\ominus}$,

$R_3$ Wasserstoff, Chlor, Brom, Fluor, Alkyl oder Alkoxy mit jeweils 1 bis 4 C-Atomen,
X″ 1,4-Phenylen, 4,4′-Biphenylen oder 2,6-Naphthylen und
R′ Alkyl mit 1 bis 4 C-Atomen, Cyclohexyl oder Phenyl bedeuten, insbesondere solche der Formel

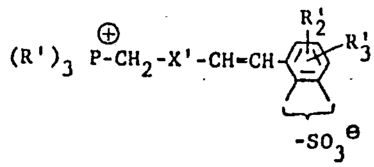

(6)

worin R′ Alkyl mit 1 bis 4 C-Atomen, Cyclohexyl oder Phenyl,
$R_2'$ und $R_3'$ unabhängig voneinander jeweils Wasserstoff, Chlor, Methyl, Methoxy oder Aethoxy und
X′ 1,4-Phenylen oder 4,4′-Biphenylen bedeuten, vor allem solche der Formel

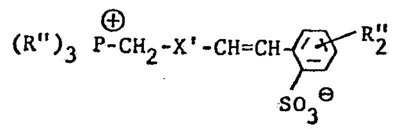

(7)

worin R″ Isopropyl, n-Butyl, Phenyl oder Cyclohexyl,
$R_2''$ Wasserstoff oder Chlor und
X′ 1,4-Phenylen oder 4,4′-Biphenylen bedeuten, und der Formel

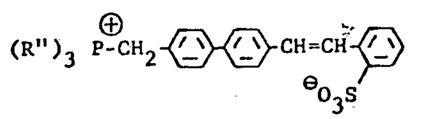

(8)

worin R″ Isopropyl, n-Butyl, Phenyl oder Cyclohexyl bedeutet.

Die Phosphoniumverbindungen der Formeln (1) bis (8) werden nach einem neuen Verfahren hergestellt, das ebenfalls Gegenstand der Erfindung ist

Dieses Verfahren zur Herstellung der Verbindungen der Formel (1) ist dadurch gekennzeichnet, dass man eine Phosphoniumverbindung der Formel

$$(R)_3 \overset{\oplus}{P}—CH_2—X—CH_2—\overset{\oplus}{P} (R)_3 \quad Q^{\ominus} \quad Q^{\ominus} \qquad (9)$$

worin R und X die in Formel (1) angegebene Bedeutung haben und $Q^{\ominus}$ ein Anion bedeutet, in Gegenwart einer stark basischen Verbindung und eines polaren Lösungsmittels mit einem Aldehyd der Formel

$$M^{\oplus}Y^{\ominus}—R_1—CHO \qquad (10)$$

worin $R_1$ und $Y^{\ominus}$ die in Formel (1) angegebene Bedeutung haben und $M^{\oplus}$ ein Kation bedeutet, umsetzt.

Für die Umsetzung ist wie erwähnt eine stark basische Verbindung erforderlich. Als solche kommen insbesondere Alkalimetallalkoholate vor allem des Kaliums und Natriums in Betracht.

Als polare Lösungsmittel kommen z.B. aprotische, wie N,N-disubstituierte Säureamide, Sulfoxide, Sulfone, Nitrile und N,N,N′,N′-tetrasubstituierte Harnstoffe oder protische Lösungsmittel wie z.B. Carbonsäuren ein- oder mehrwertige, primäre, sekundäre oder tertiäre Alkohole, vorzugsweise aliphatische, einwertige primäre Alkohole wie etwa Methanol oder Aethanol in Betracht.

Als System basische Verbindung/Lösungsmittel verwendet man daher vorzugsweise eine Lösung eines Alkalimetallalkoholats im entsprechenden Alkohol, die z.B. durch Lösen Na bzw. K in Methanol oder Aethanol erhalten wird.

Als Kationen $M^{\oplus}$ kommen neben Wasserstoff im allgemeinen Ionen von Erdalkalimetallen, z.B. von Ca, Ba oder Mg sowie insbesondere von Alkalimetallen, z.B. von Na oder K, aber auch Ammoniumionen ($NH_4^{\oplus}$) oder Aminsalzionen in Betracht. Von den Aminosalzionen sind solche bevorzugt, die sich von Mono-, Di- oder Trialkylaminen ableiten, wobei die Alkylreste auch durch Hydroxy, Cyano oder Chlor substituiert sein können. Aber auch Aminsalzionen von cyclischen Aminen wie Pyridin, Morpholin oder Piperidin kommen in Frage. Bevorzugt bedeutet $M^{\oplus}$ ein Wasserstoff-, Kalium-, Natrium- oder Ammoniumion.

Als Anionen $Q^{\ominus}$ kommen insbesondere Anionen organischer oder anorganischer Säuren, z.B. der Ameisensäure, Essigsäure, Milchsäure, einer Halogenwasserstoffsäure, Schwefelsäure, Kohlensäure sowie Monoalkylester der Schwefelsäure oder Arylsulfonsäure, wie Toluolsulfonsäure, Benzolsulfonsäure oder Halogenbenzolsulfonsäure in Betracht. $Q^{\ominus}$ bedeutet vorzugsweise ein Chlorid- oder Bromidion.

Die Reaktion der Phosphoniumverbindungen der Formel (9) mit den Aldehyden der Formel (10)

erfolgt im allgemeinen bei Temperaturen zwischen 20 und 150°C, insbesondere zwischen 40 und 140°C, vorzugsweise zwischen 40 und 100°C.

Die Phosphoniumverbindungen der Formeln (2) bis (8) werden durch Umsetzung der entsprechend substituierten Bisphosphoniumverbindungen vom Typ der Formel (9) mit den entsprechend substituierten Aldehyden vom Typ der Formel (10) nach der vorstehend beschriebenen Methode erhalten, wobei die allgemeinen Symbole jeweils die in den Formeln (2) bis (8) angegebenen Bedeutungen haben.

Die als Ausgangsstoffe verwendbaren Bis-phosphoniumverbindungen der Formel (9) werden nach an sich bekannter Weise durch Umsetzung eines Mols einer Dihalogenverbindung der Formel

$$Hal—H_2C—X—CH_2—Hal \qquad (11)$$

worin Hal Halogen, wie Chlor oder Brom bedeutet und X die oben angegebene Bedeutung hat, mit 2 Mol eines Phosphins der Formel

$$P(R)_3 \qquad (12)$$

worin R die oben angegebene Bedeutung hat, hergestellt [vgl. A. Merker, Organic Reactions *14*, 270 ff (1965) und Drefahl, Plötner und Rudolph, Chem. Ber. *93*, 998 (1960)].

Die Ausgangsverbindungen der Formel (11) sind bekannt oder können nach üblichen Verfahren leicht durch Chlormethylierung von Benzol, 4,4'-Biphenyl oder Naphthalin hergestellt werden.

Die Phosphine der Formel (12) sind bekannt.

Die Aldehyde der Formel (10) sind ebenfalls bekannt oder können nach in der Literatur beschriebenen Verfahren leicht erhalten werden.

Die erfindungsgemässen Phosphoniumverbindungen der Formeln (2) bis (8) sind wertvolle Zwischenprodukte für die Herstellung von unsymmetrisch substituierten optischen Aufhellern vom Typus

$$Z—CH{=}CH—X—CH{=}CH—R_1—Y \qquad (13)$$

worin X, Y und $R_1$ wie in Formel (1) definiert sind und Z einen aromatischen Rest bedeutet. Derartige Verbindungen sind z.B. in den U.S. Patentschriften 4,008,224, 3,984,399, 3,843,633 und 3,849,485 beschrieben. Gemäss diesen Patentschriften mussten solche unsymmetrisch substituierten Aufheller bisher durch eine Mischkondensation, wobei eine Bis-phosphoniumverbindung mit einem Gemisch von zwei verschiedenen Aldehyden umgesetzt wurde, hergestellt werden. Diese Methode führte naturgemäss zu geringen Ausbeuten und nicht sehr reinen Produkten, da immer auch symmetrische Verbindungen als Nebenprodukte entstanden.

Ueberraschenderweise kann man nun mit Hilfe eines neuen Verfahrens, in dem die erfindungsgemässen Verbindungen der Formel (1) als Zwischenprodukte auftreten und das ebenfalls Gegenstand der Erfindung ist, die erwähnten unsymmetrisch substituierten Aufheller selektiv und ohne das Auftreten von symmetrischen Nebenprodukten und damit in guten Ausbeuten synthetisieren.

Das erfindungsgemässe Verfahren zur Herstellung von unsymmetrisch substituierten optischen Aufhellern der Formel

$$Z—CH{=}CH—X—CH{=}CH—R_1—Y^{\ominus}\ M^{\oplus} \qquad (14)$$

worin $R_1$ einen gegebenenfalls neben der Gruppe $Y^{\ominus}$ weitere nicht-chromophore Substituenten aufweisenden Phenyl-, Naphthyl-, Furan-, Thiophen-, Oxazol-, Isoxazol-, Pyrazol-, Imidazol- Triazol- oder Oxadiazolrest, wobei zwei Substituenten in ortho-Stellung auch die Ergänzung zu einem unsubstituierten oder nicht-chromophor substituierten Benzo- oder Naphthorest bilden können, und
X Phenylen, 4,4'-Biphenylen oder 1,4- oder 2,6-Naphthylen bedeuten,
Z die gleichen Bedeutungsmöglichkeiten wie $R_1$ hat, wobei Z jedoch von der Gruppe $—R_1—Y^{\ominus}\ M^{\oplus}$ verschieden ist,
$Y^{\ominus}$ $SO_3^{\ominus}$, $SO_2^{\ominus}$ oder $COO^{\ominus}$ und
$M^{\oplus}$ ein Kation bedeuten, ist dadurch gekennzeichnet, dass man eine Phosphoniumverbindung der Formel

$$(R)_3\ \overset{\oplus}{P}—CH_2—X—CH_2—\overset{\oplus}{P}\ (R)_3 \qquad (9)$$
$$Q^{\ominus} \qquad\qquad Q^{\ominus}$$

worin X die vorstehend angegebene Bedeutung hat, R Alkyl mit 1 bis 6 C-Atomen, Cycloalkyl mit 5 bis 7 C-Atomen, $—NX_1X_2$, worin $X_1$ und $X_2$ unabhängig voneinander für Alkyl mit 1 bis 4 C-Atomen oder zusammen mit dem N-Atom für einen 5- oder 6-gliedrigen gesättigten heterocyclischen Ring stehen; oder unsubstituiertes oder durch Alkyl mit 1—6 C-Atomen, Alkoxy mit 1—6 C-Atomen, Chlor, Fluor, Brom, Jod, Cycloalkyl mit 5—7 Ringgliedern, Phenyl, Phenoxy, Naphthoxy oder durch die Gruppe $—NX_1X_2$, worin $X_1$ und $X_2$ wie vorstehend definiert sind, substituiertes Phenyl und $Q^{\ominus}$ ein Anion bedeuten, in Gegenwart einer stark basischen Verbindung in einem polaren Lösungsmittel mit einem

Aldehyd der Formel

$$M^{\oplus}\ Y^{\ominus}—R_1—CHO \qquad (10)$$

worin $R_1$, $Y^{\ominus}$ und $M^{\oplus}$ wie oben definiert sind, bei Temperaturen zwischen 20 und 150°C umsetzt und das erhaltene Reaktionsprodukt der Formel

$$(R)_3\overset{\oplus}{P}—CH_2—X—CH{=}CH—R_1—Y^{\ominus} \qquad (1)$$

nach dessen Isolierung oder direkt in der Reaktionsmischung mit einem Aldehyd der Formel Z—CHO bei Temperaturen von 100 bis 200°C in Gegenwart einer stark basischen Verbindung in einem bei über 100°C siedendem polaren Lösungsmittel umsetzt.

Nähere Erklärungen zu den allgemeinen Symbolen in Formel (14) sind bei den vorstehenden Erläuterungen zu den Formeln (1), (9) und (10) zu finden.

Die Verfahrensparameter für die erste Stufe entsprechen jenen für das Verfahren zur Herstellung der Verbindungen der Formel (1). Die dort angegebenen Erläuterungen gelten daher auch hier.

Das Zwischenprodukt der Formel (1) kann isoliert werden, es kann aber auch ohne Isolierung direkt in der Reaktionsmischung mit dem Aldehyd Z—CHO weiter umgesetzt werden, gegebenenfalls nach Abdampfen des Lösungsmittels. Vorzugsweise wird das Zwischenprodukt jedoch isoliert.

Für die zweite Stufe, d.h. die Umsetzung des Zwischenproduktes der Formel (1) mit dem Aldehyd Z—CHO ist ebenfalls eine stark basische Verbindung erforderlich. Zu erwähnen sind hierbei Alkalimetall- und Erdalkalimetallhydroxide, z.B. KOH, NaOH sowie Alkalimetallalkoholate, metallisches Natrium oder Kalium in entsprechenden Lösungsmittel.

Als polare Lösungsmittel kommen solche mit einem Siedepunkt von über 100°C in Frage, z.B. aprotische wie N,N-disubstituierte Säureamide, Sulfoxide, Sulfone, Nitrile und N,N,N',N'-tetrasubstituierte Harnstoffe, oder protische Lösungsmittel, wie z.B. Carbonsäuren oder höher siedende Alkohole, z.B. tertiäre, insbesondere sekundäre Alkohole. Aethylenglykol ist ein bevorzugtes Lösungsmittel.

Im Rahmen des erfindungsgemässen Verfahrens ist besonders zu erwähnen die Herstellung von Verbindungen der Formeln

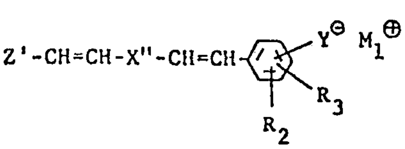 (15) und 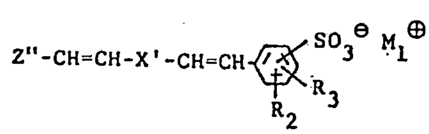 (16)

worin $M_1^{\oplus}$ ein Wasserstoff-, Alkalimetall-, Erdalkalimetall-, Ammonium- oder Aminion,
$Y^{\ominus}$ —$SO_3^{\ominus}$, —$SO_2^{\ominus}$ oder —$COO^{\ominus}$
$R_2$ Wasserstoff, Chlor, Brom, Fluor, Alkyl oder Alkoxy mit jeweils 1 bis 4 C-Atomen oder die Gruppe $Y^{\ominus}$ $M_1^{\oplus}$,
$R_3$ Wasserstoff, Chlor, Brom, Fluor oder Alkyl oder Alkoxy mit jeweils 1 bis 4 C-Atomen,
X'' 1,4-Phenylen, 4,4'-Biphenylen oder 2,6-Naphthylen,
Z' eine Gruppe der Formel

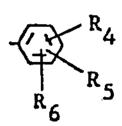 oder 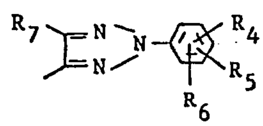

worin $R_4$ Wasserstoff, Chlor, Brom, Fluor, Alkyl oder Alkoxy mit jeweils 1 bis 4 C-Atomen, Cyano oder zusammen mit $R_5$ in ortho-Stellung den Rest —O—$CH_2$—O— oder —O—$CH_2$—O—$CH_2$—,
$R_5$ Wasserstoff, Chlor, Brom, Fluor, Alkyl oder Alkoxy mit jeweils 1 bis 4 C-Atomen oder zusammen mit $R_4$ in ortho-Stellung den Rest —O—$CH_2$—O— oder —O—$CH_2$—O—$CH_2$—,
$R_6$ Wasserstoff, Chlor, Brom, Fluor oder Alkyl mit 1 bis 4 C-Atomen und
$R_7$ Wasserstoff, Alkyl mit 1 bis 4 C-Atomen, Chlor oder Phenyl darstellen,
X' 1,4-Phenylen oder 4,4'-Biphenylen und
Z'' eine Gruppe der Formel

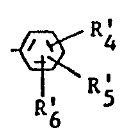 oder 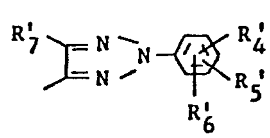

bedeuten,
worin $R_4'$ Wasserstoff, Chlor, Methyl, Methoxy oder Aethoxy oder gemeinsam mit $R_5'$ in ortho-Stellung den Rest —O—$CH_2$—O— oder —O—$CH_2$—O—$CH_2$—,
$R_5'$ Wasserstoff, Chlor, Methyl, Methoxy oder Aethoxy oder gemeinsam mit $R_4'$ in ortho-Stellung den Rest —O—$CH_2$—O— oder —O—$CH_2$—O—$CH_2$—,

$R_6'$ Wasserstoff, Chlor oder Methyl und
$R_7'$ Wasserstoff, Methyl oder Aethyl darstellen, durch Umsetzung einer Bis-phosphoniumverbindung der Formel

$$(R')_3\overset{\oplus}{P}—CH_2—X''—CH_2—\overset{\oplus}{P}(R')_3 \quad Q^{\ominus} \quad Q^{\ominus} \qquad (17)\text{ bzw.}$$

$$(R')_3\overset{\oplus}{P}—CH_2—X'—CH_2—\overset{\oplus}{P}(R')_3 \quad Q^{\ominus} \quad Q^{\ominus} \qquad (18)$$

mit einem Aldehyd der Formel

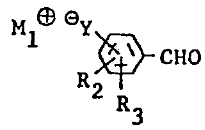

(19)

und Umsetzung des erhaltenen Zwischenproduktes mit einem Aldehyd der Formel Z'—CHO bzw. Z'' — CHO.

Bevorzugt ist die Herstellung von Verbindungen der Formel

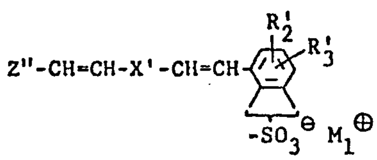

(20) bzw.

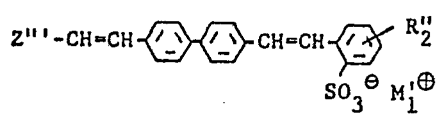

(21)

worin X', Z'' und $M_1^{\oplus}$ wie in Formel (16) definiert sind.
$R_2'$ und $R_3'$ unabhängig voneinander jeweils Wasserstoff, Chlor, Methyl, Methoxy oder Aethoxy,
$R_2''$ Wasserstoff oder Chlor,
$M_1'^{\oplus}$ ein Wasserstoff-, Natrium-, Kalium- oder Ammoniumion und
Z''' einen Rest der Formel

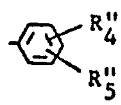

oder

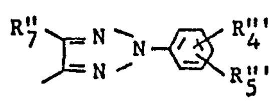

bedeuten, worin $R_4''$ Wasserstoff, Chlor, Methyl, Methoxy oder zusammen mit $R_5''$ in ortho-Stellung die Gruppe der Formel —O—$CH_2$—O—$CH_2$—,
$R_5''$ Wasserstoff, Chlor, Methyl oder zusammen mit $R_4''$ in ortho-Stellung die Gruppe der Formel —O—$CH_2$—O—$CH_2$—,
$R_4'''$ Wasserstoff, Chlor, Methyl, Methoxy oder Aethoxy,
$R_5'''$ Wasserstoff, Chlor oder Methyl und
$R_7''$ Wasserstoff oder Methyl darstellen, durch Umsetzung einer Bis-phosphoniumverbindung der Formel

$$(R')_3\overset{\oplus}{P}—CH_2—X'—CH_2—\overset{\oplus}{P}(R')_3 \quad Q^{\ominus} \quad Q^{\ominus} \qquad (18)\text{ bzw.}$$

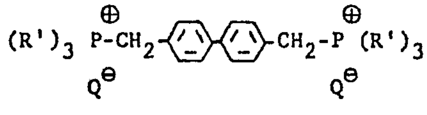

(22)

mit einem Aldehyd der Formel

(23) bzw.

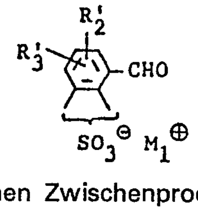

(24)

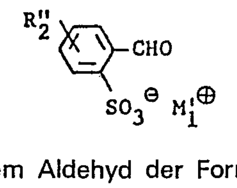

und Umsetzung des erhaltenen Zwischenproduktes mit einem Aldehyd der Formel Z''—CHO bzw. Z'''—CHO.

Ersetzt man in Formel (22) den Biphenylenrest durch einen Phenylenrest, kommt man zu ebenfalls bevorzugten Aufhellern der Formel (21) mit einem Phenylrest anstelle des Biphenylenrestes.

Die folgenden Beispiele veranschaulichen die Erfindung, ohne sie auf diese Beispiele zu beschränken. Wenn nicht anders vermerkt, bedeuten Prozent Gewichtsprozent und Teile Gewichtsteile.

Beispiel 1

In einem 200 ml Kolben werden unter Rühren in Stickstoffatmosphäre 0,253 g metallisches Natrium portionenweise in 50 ml wasserfreiem Methanol gelöst. Danach wird die Alkoholatlösung mit 6,56 g der Phosphoniumverbindung der Formel

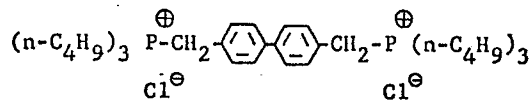
(100)

und 2,53 g Natriumsalz der Benzaldehyd-2-sulfonsäure (91%ig) versetzt. Die Reaktionsmischung wird auf 40 bis 45°C erwärmt und 16 Stunden lang gerührt. Die entstandene weisse Suspension wird mit 80 ml Wasser versetzt und während 1 Stunde bei Siedetemperatur gerührt. Die weisse Suspension wird nun abgenutscht und mit viel siedendem Wasser gewaschen und im Vakuum bei 100°C während 24 Stunden getrocknet. Man erhält 5,48 g (98% der Theorie) der Verbindung der Formel

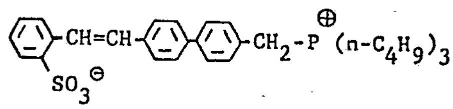
(101)

mit einem $\lambda_{max}$ 335 nm in Dimethylformamid.

Analyse:

| | | | | |
|---|---|---|---|---|
| Berechnet: | C 71,97 | H 7,87 | S 5,82 | P 5,62 |
| Gefunden: | C 71,3 | H 7,6 | S 5,9 | P 5,6 |

Verwendet man anstelle der Verbindung der Formel (100) die Verbindungen der Formeln

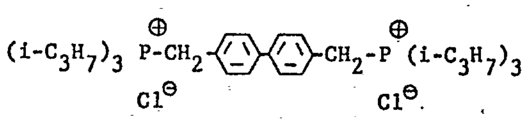
(102)

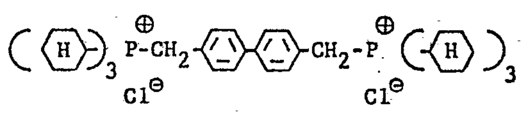
(103)

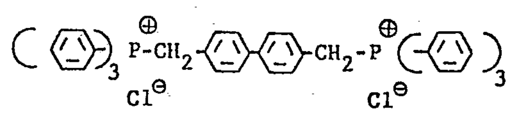
(104)

und verfährt ansonst wie im Beispiel 1 beschrieben, so erhält man die Verbindungen der folgenden Formeln:

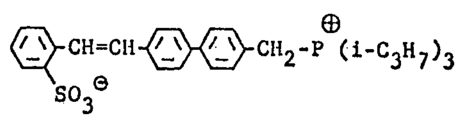
(105)

Ausbeute: 40%
$\lambda_{max} = 330$ nm in Dimethylformamid

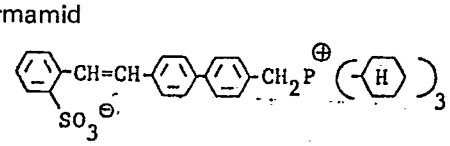
(106)

Ausbeute: 80%
$\lambda_{max} = 320$ nm in Dimethylformamid/Wasser

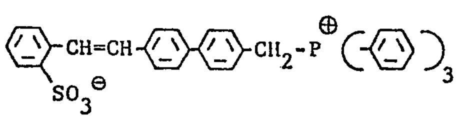
(107)

Ausbeute: 90%
$\lambda_{max} = 329$ nm in Aethanol

Bei der Verwendung der Verbindung (104) wird die Umsetzung mit dem Natriumsalz der Benzaldehyd-2-sulfonsäure in Gegenwart von molaren Mengen Natriumbisulfit vorgenommen, um die Bildung eines 1:1 cis/trans Gemisches zu verhindern. Die Verbindung der Formel (104) kann z.B. auch mit dem Natriumsalz der 2-Formyl-furan-5-sulfonsäure gemäss der im Beispiel 1 beschriebenen Methode umgesetzt werden, wobei man die Verbindung der Formel

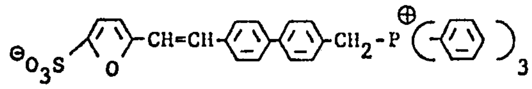

(108)

erhält.

Ausbeute: 90%
$\lambda_{max} = 350$ nm in Methyenchlorid

Beispiel 2

Wiederholt man das Verfahren gemäss Beispiel 1, ersetzt jedoch die Verbindung (100) durch die entsprechenden Bis-phosphonomethylbenzol-, -biphenyl- bzw.-naphthalinverbindungen und Benzaldehyd-2-sulfonsäure durch 5-Sulfo-furfurol, so erhält man die in Tabelle 1 angeführten Verbindungen der Formel

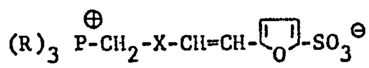

(200)

TABELLE 1

| Verbindung Nr. | R | X | Ausbeute |
|---|---|---|---|
| 201 | n-$C_4H_9$ | 4,4'-Biphenylen | 93,4 |
| 202 | n-$C_4H_9$ | 1,4-Phenylen | 88,3 |
| 203 | Phenyl | 1,4-Phenylen | 91,9 |

Beispiel 3

Wiederholt man das Verfahren gemäss Beispiel 1, ersetzt jedoch die Verbindung (100) durch die entsprechenden Bis-phosphonomethylbenzol-, -biphenyl- bzw.-naphthalinverbindungen und setzt letztere mit entsprechend substituierten Benzaldehyden um, so erhält man die in Tabelle 2 angeführten Verbindungen der Formel

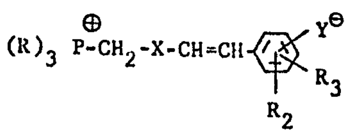

(300)

TABELLE 2

| Verbin. dung Nr. | R | X | $R_2$ | $R_3$ | $Y^{\ominus}$ |
|---|---|---|---|---|---|
| 204 | $n-C_4H_9$ | 1,4-Phenylen | H | H | $2-SO_3^{\ominus}$ |
| 205 | Phenyl | 1,4-Phenylen | H | H | $2-SO_3^{\ominus}$ |
| 206 | Cyclohexyl | 1,4-Phenylen | H | H | $2-SO_3^{\ominus}$ |
| 207 | $n-C_4H_9$ | 4,4'-Biphenylen | H | H | $2-COO^{\ominus}$ |
| 208 | $n-C_4H_9$ | 4,4'-Biphenylen | H | H | $4-COO^{\ominus}$ |
| 209 | $n-C_4H_9$ | 4,4'-Biphenylen | 4–Cl | H | $3-SO_3^{\ominus}$ |
| 210 | Cyclohexyl | 4,4'-Biphenylen | H | H | $4-SO_2^{\ominus}$ |
| 211 | $i-C_3H_7$ | 4,4'-Biphenylen | 3–Cl | H | $2-SO_3^{\ominus}$ |
| 212 | $n-C_4H_9$ | 4,4'-Biphenylen | 4–Cl | H | $2-SO_3^{\ominus}$ |
| 213 | Phenyl | 4,4'-Biphenylen | $2-SO_3H$ | H | $4-SO_3^{\ominus}$ |
| 214 | Cyclohexyl | 4,4'-Biphenylen | $3-CH_3$ | $4-CH_3$ | $2-SO_3^{\ominus}$ |
| 215 | $n-C_4H_9$ | 4,4'-Biphenylen | $4-OCH_3$ | H | $2-SO_3^{\ominus}$ |
| 216 | $CH_3$ | 4,4'-Biphenylen | H | H | $2-COO^{\ominus}$ |
| 217 | Phenyl | 4,4'-Biphenylen | 3–Cl | $4-CH_3$ | $2-SO_3^{\ominus}$ |
| 218 | $n-C_4H_9$ | 4,4'-Biphenylen | 2–Cl | H | $3-SO_3^{\ominus}$ |
| 219 | $n-C_4H_9$ | 4,4'-Biphenylen | $2-CH_3$ | H | $3-SO_3^{\ominus}$ |
| 220 | $n-C_4H_9$ | 1,4-Phenylen | $3-CH_3$ | H | $4-COO^{\ominus}$ |
| 221 | $n-C_4H_9$ | 1,4-Naphthylen | H | H | $2-SO_3^{\ominus}$ |
| 222 | $n-C_4H_9$ | 2,6-Naphthylen | H | H | $2-SO_3^{\ominus}$ |

## Beispiel 4

In einem 200 ml Kolben werden 5,5 ml 2N NaOH in Aethylenglykol und 45 ml Aethylenglykol unter Stickstoffatmosphäre vorgelegt. Dieser Lösung werden 5,58 g der Verbindung (101) und 2,22 g des Bisulfitadduktes des Aldehyds der Formel

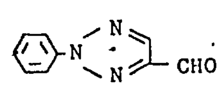

(400)

zugegeben. Die entstandene weisse Suspension wird auf 140°C erwärmt und 4 Stunden lang gerührt. Man destilliert unter Vakuum das Aethylenglykol ab, versetzt den Rückstand mit 100 ml eines Wasser/Dimethylformamid-Gemisches (9:1) und erhitzt das Ganze auf Siedetemperatur. Die heisse Lösung wird filtriert unter Zuhilfenahme von Bleicherde und mit 50 ml Wasser/Dimethylformamid (9:1) nachgewaschen. Das Filtrat wird zum Sieden erhitzt, bis alles gelöst ist. Anschliessend werden 14 g NaCl zugesetzt und man lässt erkalten. Das gebildete grüngelbe Produkt wird abgenutscht, mit 15 ml 10%iger NaCl-Lösung nachgewaschen und während 24 Stunden im Vakuum bei 100°C getrocknet. Man erhält 4,53 g (86% der Theorie) des Aufhellers der Formel

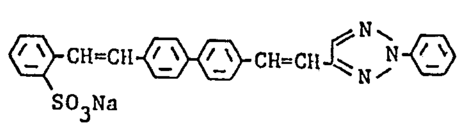

(401)

Wiederholt man die vorstehende Herstellungsvorschrift, setzt jedoch anstelle der Verbindung (101) die entsprechende in Beispiel 1 oder 3 beschriebene Verbindung und anstelle des Aldehyds (400) einen entsprechend substituierten Aldehyd ein, so erhält man die in Tabelle 3 angeführten Verbindungen der Formel

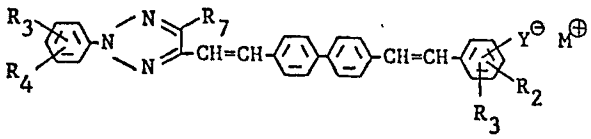

(402)

M = Na, K oder H

TABELLE 3

| Verbindung Nr. | $R_3$ | $R_4$ | $R_7$ | $R_2$ | $R_3$ | $Y^{\ominus}$ |
|---|---|---|---|---|---|---|
| 403 | H | H | $CH_3$ | H | H | $2-SO_3^{\ominus}$ |
| 404 | 3–Cl | H | H | H | H | $2-SO_3^{\ominus}$ |
| 405 | $4-CH_3$ | H | H | H | H | $2-SO_3^{\ominus}$ |
| 406 | H | H | H | 3–Cl | H | $2-SO_3^{\ominus}$ |
| 407 | H | H | H | 4–Cl | H | $2-SO_3^{\ominus}$ |
| 408 | $3-CH_3$ | H | H | H | H | $2-SO_3^{\ominus}$ |
| 409 | $3-OCH_3$ | H | H | H | H | $2-SO_3^{\ominus}$ |
| 410 | 3–F | H | H | H | H | $2-SO_3^{\ominus}$ |
| 411 | 4–F | H | H | H | H | $2-SO_3^{\ominus}$ |
| 412 | 4–Cl | H | H | H | H | $2-SO_3^{\ominus}$ |
| 413 | $3-CH_3$ | 4–Cl | H | H | H | $2-SO_3^{\ominus}$ |
| 414 | 3–Br | H | H | H | H | $2-SO_3^{\ominus}$ |
| 415 | 3–Br | 4–Br | H | H | H | $2-SO_3^{\ominus}$ |
| 416 | $2-CH_3$ | 4–Br | H | H | H | $2-SO_3^{\ominus}$ |
| 417 | $4-C_2H_5$ | H | H | H | H | $2-SO_3^{\ominus}$ |
| 418 | $3-CH_3$ | $4-CH_3$ | H | H | H | $2-SO_3^{\ominus}$ |
| 419 | $3,4-O-CH_2-O-CH_2-$ | | H | H | H | $4-SO_3^{\ominus}$ |
| 420 | $3,4-O-CH_2-O-$ | | H | H | H | $3-SO_3^{\ominus}$ |
| 421 | H | H | $C_6H_5$ | H | H | $2-SO_3^{\ominus}$ |
| 422 | H | H | H | $2-SO_3Na$ | H | $4-SO_3^{\ominus}$ |
| 423 | H | H | H | $3-CH_3$ | $4-CH_3$ | $2-SO_3^{\ominus}$ |
| 424 | H | H | H | $4-OCH_3$ | H | $2-SO_3^{\ominus}$ |
| 425 | H | H | H | H | H | $4-COO^{\ominus}$ |
| 426 | 3–Cl | H | H | 3–Cl | $4-CH_3$ | $2-SO_3^{\ominus}$ |

Beispiel 5

0,25 g Natrium (0,011 Mol) werden bei 40 bis 45°C in 100 ml Aethylenglykol gelöst und anschliessend werden zur klaren Lösung unter Stickstoffatmosphäre 5,5 g (0,01 Mol) der Verbindung der Formel (101) und 1,16 g (0,011 Mol) Benzaldehyd unter Rühren zugesetzt. Das Gemisch wird auf 140°C erwärmt und über Nacht gerührt, wobei eine gelbgrüne Suspension entsteht. Das Reaktionsgemisch wird dann auf ca. 80°C abgekühlt und das Lösungsmittel am Wasserstrahlvakuum abdestilliert. Der Rückstand wird dreimal in 50 ml Toluol digeriert und gekocht, um Reste von Aldehyd und Tributylphosphinoxid zu entfernen. Der nun feste Rückstand wird in 100 ml Wasser/Dimethylformamid 9:1 aufgekocht, die siedende Lösung filtriert, das Filtrat wieder zum Sieden gebracht und mit 10 g NaCl versetzt. Man lässt die gelbliche Suspension unter Rühren abkühlen, rührt noch etwa 3 Stunden bei

Raumtemperatur, filtriert das Produkt ab, wäscht mit wenig 10% NaCl-Lösung nach und trocknet das Produkt bei 120°C im Vakuum. Man erhält so etwa 4 g des Aufhellers der Formel

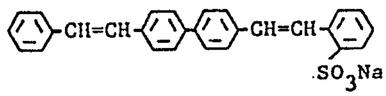

(500)

Unter Verwendung der entsprechenden gemäss den Beispielen 1 und 3 erhaltenen Phosphoniumverbindungen und der entsprechend substituierten Aldehyde erhält man in analoger Weise die in Tabelle 4 angeführten Verbindungen der Formel

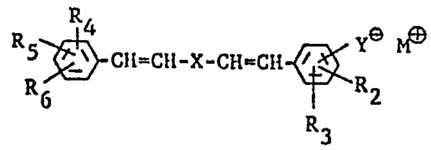

(501)

M = Na, K oder H

TABELLE 4

| Verbindung Nr. | $R_4$ | $R_5$ | $R_6$ | X | $R_2$ | $R_3$ | $Y^{\ominus}$ |
|---|---|---|---|---|---|---|---|
| 502 | $2-CH_3$ | H | H | 4,4'-Biphenylen | H | H | $2-SO_3^{\ominus}$ |
| 503 | 2-Cl | H | H | 4,4'-Biphenylen | H | H | $2-SO_3^{\ominus}$ |
| 504 | $4-OCH_3$ | H | H | 4,4'-Biphenylen | H | H | $2-SO_3^{\ominus}$ |
| 505 | 3-Cl | H | H | 4,4'-Biphenylen | H | H | $2-SO_3^{\ominus}$ |
| 506 | $4-CH_3$ | H | H | 4,4'-Biphenylen | H | H | $2-SO_3^{\ominus}$ |
| 507 | $3,4-O-CH_2-O-CH_2-$ | | H | 4,4'-Biphenylen | H | H | $2-SO_3^{\ominus}$ |
| 508 | $3,4-O-CH_2-O-$ | | H | 4,4'-Biphenylen | H | H | $2-SO_3^{\ominus}$ |
| 509 | 3-CN | H | H | 4,4'-Biphenylen | H | H | $2-SO_3^{\ominus}$ |
| 510 | 2-Cl | 4-Cl | H | 4,4'-Biphenylen | H | H | $2-SO_3^{\ominus}$ |
| 511 | $2-OCH_3$ | H | H | 4,4'-Biphenylen | H | H | $2-SO_3^{\ominus}$ |
| 512 | 2-Cl | 6-Cl | H | 4,4'-Biphenylen | H | H | $2-SO_3^{\ominus}$ |
| 513 | $2-CH_3$ | $4-CH_3$ | 6-Cl | 4,4'-Biphenylen | H | H | $2-SO_3^{\ominus}$ |
| 514 | $2-OCH_3$ | $3-OCH_3$ | H | 4,4'-Biphenylen | H | H | $2-SO_3^{\ominus}$ |
| 515 | 3-F | H | H | 4,4'-Biphenylen | H | H | $2-SO_3^{\ominus}$ |
| 516 | $4-t-C_4H_9$ | H | H | 4,4'-Biphenylen | H | H | $2-SO_3^{\ominus}$ |
| 517 | 3-Cl | $4-CH_3$ | H | 4,4'-Biphenylen | H | H | $2-SO_3^{\ominus}$ |
| 518 | $2-C_2H_5$ | $4-C_2H_5$ | H | 4,4'-Biphenylen | H | H | $2-SO_3^{\ominus}$ |
| 519 | H | H | H | 4,4'-Biphenylen | 2-Cl | H | $3-SO_3^{\ominus}$ |
| 520 | 2-Cl | H | H | 4,4'-Biphenylen | $2-CH_3$ | H | $3-SO_3^{\ominus}$ |
| 521 | $3-OCH_3$ | H | H | 4,4'-Biphenylen | 4-Cl | H | $3-SO_3^{\ominus}$ |
| 522 | H | H | H | 4,4'-Biphenylen | 3-Cl | $4-CH_3$ | $2-SO_3^{\ominus}$ |
| 523 | H | H | H | 4,4'-Biphenylen | H | H | $2-SO_2^{\ominus}$ |
| 524 | H | H | H | 4,4'-Biphenylen | $3-CH_3$ | H | $4-COO^{\ominus}$ |
| 525 | $2-OCH_3$ | H | H | 1,4-Phenylen | H | H | $2-SO_3^{\ominus}$ |
| 526 | $2-OCH_3$ | $3-OCH_3$ | H | 1,4-Phenylen | H | H | $2-SO_3^{\ominus}$ |
| 527 | 4-Cl | H | H | 1,4-Phenylen | H | H | $2-SO_3^{\ominus}$ |
| 528 | 2-Cl | 4-Cl | H | 1,4-Phenylen | H | H | $2-SO_3^{\ominus}$ |
| 529 | $2-CH_3$ | H | H | 1,4-Phenylen | H | H | $2-SO_3^{\ominus}$ |
| 530 | 3-Cl | H | H | 1,4-Phenylen | $2-SO_3Na$ | H | $4-SO_3^{\ominus}$ |
| 531 | H | H | H | 1,4-Naphthylen | H | H | $2-SO_3^{\ominus}$ |
| 532 | H | H | H | 2,6-Naphthylen | H | H | $2-SO_3^{\ominus}$ |

## Patentansprüche

1. Phosphoniumverbindungen der Formel

$$(R_3)\overset{\oplus}{P}—CH_2—X—CH=CH—R_1—Y^{\ominus}$$

worin R Alkyl mit 1 bis 6 C-Atomen, Cycloalkyl mit 5 bis 7 C-Atomen, $—NX_1X_2$, worin $X_1$ und $X_2$ unabhängig voneinander für Alkyl mit 1 bis 4 C-Atomen oder zusammen mit dem N-Atom für einen 5- oder 6-gliedrigen gesättigten heterocyclischen Ring stehen; oder unsubstituiertes oder durch Alkyl mit 1—6 C-Atomen, Alkoxy mit 1—6 C-Atomen, Chlor, Fluor, Brom, Jod, Cycloalkyl mit 5—7 Ringgliedern, Phenyl, Phenoxy, Naphthoxy oder durch die Gruppe $—NX_1X_2$, worin $X_1$ und $X_2$ wie vorstehend definiert sind, substituiertes Phenyl,
$R_1$ einen gegebenenfalls neben der Gruppe $Y^{\ominus}$ weitere nicht-chromophore Substituenten aufweisenden Phenyl-, Naphthyl-, Furan-, Thiophen-, Oxazol-, Isoxazol-, Pyrazol-, Imidazol-, Triazol- oder Oxadiazolrest, wobei zwei Substituenten in ortho-Stellung auch die Ergänzung zu einem unsubstituierten oder nicht-chromophor substituierten Benzo- oder Naphthorest bilden können.
X Phenylen, 4,4'-Biphenylen oder 1,4- oder 2,6-Naphthylen und $Y^{\ominus}$ $SO_3^{\ominus}$, $SO_2^{\ominus}$ oder $COO^{\ominus}$ bedeuten.

2. Phosphoniumverbindungen gemäss Anspruch 1 der Formel

$$(R')_3\overset{\oplus}{P}—CH_2—X''—CH=CH—R_1'—Y^{\ominus}$$

worin $R_1'$ einen gegebenenfalls neben der Gruppe $Y^{\ominus}$ einen oder zwei weitere nicht-chromophore Substituenten tragenden Phenyl-, Naphthyl-, Furan-, Thiophen-, Oxazol-, Isoxazol-, Pyrazol-, Imidazol-, Triazol- oder Oxadiazolrest, wobei zwei benachbarte Substituenten auch die Ergänzung zu einem ankondensierten Benzo- oder Naphthorest bilden können.
R' Alkyl mit 1 bis 4 C-Atomen, Cyclohexyl oder Phenyl,
$Y^{\ominus}$ $SO_3^{\ominus}$, $SO_2^{\ominus}$ oder $COO^{\ominus}$ und
X'' 1,4-Phenylen, 2,6-Naphthylen oder 4,4'-Biphenylen bedeuten.

3. Phosphoniumverbindungen gemäss Anspruch 2 der Formel

$$(R')_3\overset{\oplus}{P}—CH_2—X'—CH=CH—R_1''—Y_1^{\ominus}$$

worin $R_1''$ einen gegebenenfalls neben der Gruppe $Y_1^{\ominus}$ einen oder zwei weitere Substituenten aus der Gruppe Alkyl mit 1 bis 4 C-Atomen, Alkoxy mit 1 bis 4 C-Atomen, Cyclohexyl, Alkenyl mit 3 oder 4 C-Atomen, Phenyl, Sulfo, Fluor, Chlor, Brom oder Carbalkoxy mit 2 bis 5 C-Atomen tragenden Phenyl- oder Furanrest, R' Alkyl mit 1 bis 4 C-Atomen, Cyclohexyl oder Phenyl,
$Y_1^{\ominus}$ $SO_3^{\ominus}$ oder $COO^{\ominus}$ und
X' 1,4-Phenylen oder 4,4'-Biphenylen bedeuten

4. Phosphoniumverbindungen gemäss Anspruch 1 der Formel

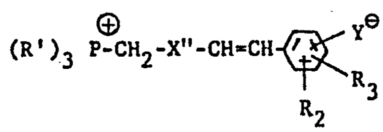

worin $Y^{\ominus}$ $—SO_3^{\ominus}$, $—SO_2^{\ominus}$ oder $—COO^{\ominus}$,
$R_2$ Wasserstoff, Chlor, Brom, Fluor, Alkyl oder Alkoxy mit jeweils 1 bis 4 C-Atomen oder eine weitere Gruppe $Y^{\ominus}$,
$R_3$ Wasserstoff, Chlor, Brom, Fluor, Alkyl oder Alkoxy mit jeweils 1 bis 4 C-Atomen,
X'' 1,4-Phenylen, 4,4'-Biphenylen oder 2,6-Naphthylen und
R' Alkyl mit 1 bis 4 C-Atomen, Cyclohexyl oder Phenyl bedeuten.

5. Phosphoniumverbindungen gemäss Anspruch 4 der Formel

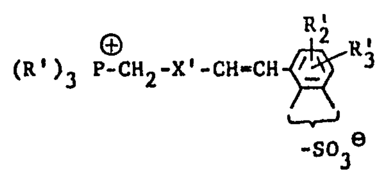

worin R' Alkyl mit 1 bis 4 C-Atomen, Cyclohexyl oder Phenyl,
$R_2'$ und $R_3'$ unabhängig voneinander jeweils Wasserstoff, Chlor, Methyl, Methoxy oder Aethoxy und
X' 1,4-Phenylen oder 4,4'-Biphenylen bedeuten.

6. Verfahren zur Herstellung von Phosphoniumverbindungen der im Anspruch 1 angegebenen

Formel, dadurch gekennzeichnet, dass man eine Phosphoniumverbindung der Formel

$$(R)_3\overset{\oplus}{P}—CH_2—X—CH_2—\overset{\oplus}{P}(R)_3 \quad Q^{\ominus} \quad Q^{\ominus}$$

worin R und X wie in Anspruch 1 definiert sind und $Q^{\ominus}$ ein Anion bedeutet, in Gegenwart einer stark basischen Verbindung und eines polaren Lösungsmittels mit einem Aldehyd der Formel

$$M^{\oplus}Y^{\ominus}—R_1—CHO$$

worin $R_1$ und $Y^{\ominus}$ wie in Anspruch 1 definiert sind und $M^{\oplus}$ ein Kation bedeutet, umsetzt.

7. Verfahren gemäss Anspruch 6 zur Herstellung von Phosphoniumverbindungen der Formel

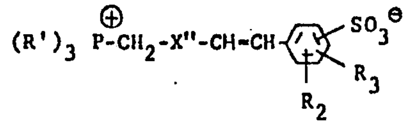

worin $R_2$ Wasserstoff, Chlor, Brom, Fluor, Alkyl oder Alkoxy mit jeweils 1 bis 4 C-Atomen oder eine Sulfogruppe,
$R_3$ Wasserstoff, Chlor, Brom, Fluor, Alkyl oder Alkoxy mit jeweils 1 bis 4 C-Atomen,
$X''$ 1,4-Phenylen, 4,4'-Biphenylen oder 2,6-Naphthylen und
$R'$ Alkyl mit 1 bis 4 C-Atomen, Cyclohexyl oder Phenyl bedeuten, durch Umsetzung einer Phosphoniumverbindung der Formel

$$(R')_3\overset{\oplus}{P}—CH_2—X''—CH_2—\overset{\oplus}{P}(R')_3 \quad Q_1^{\ominus} \quad Q_1^{\ominus}$$

worin $R'$ und $X''$ die vorstehend angegebene Bedeutung haben und $Q_1^{\ominus}$ ein Chlorid- oder Bromidion bedeutet, mit einem Aldehyd der Formel

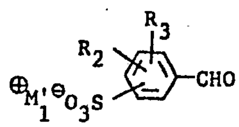

worin $R_2$ und $R_3$ die vorstehend angegebene Bedeutung haben und $M_1'^{\oplus}$ ein Wasserstoff- Natrium-, Kalium- oder Ammoniumion bedeutet.

8. Verfahren gemäss Anspruch 6, dadurch gekennzeichnet, dass man die Umsetzung in Gegenwart eines Natrium- oder Kaliumalkoholates bei Temperaturen zwischen 20 und 150°C durchführt.

9. Verfahren gemäss Anspruch 6, dadurch gekennzeichnet, dass man als polares Lösungsmittel einen Alkohol verwendet.

10. Verfahren zur Herstellung von unsymmetrischen optischen Aufhellern der Formel

$$Z—CH{=}CH—X—CH{=}CH—R_1—Y^{\ominus}\ M^{\oplus}$$

worin $R_1$ einen gegebenenfalls neben der Gruppe $Y^{\ominus}$ weitere nicht-chromophore Substituenten aufweisenden Phenyl-, Naphthyl-, Furan-, Thiophen-, Oxazol-, Isoxazol-, Pyrazol-, Imidazol- Triazol- oder Oxadiazolrest, wobei zwei Substituenten in ortho-Stellung auch die Ergänzung zu einem unsubstituierten oder nicht-chromophor substituierten Benzo- oder Naphthorest bilden können, und
X Phenylen, 4,4'-Biphenylen oder 1,4- oder 2,6-Naphthylen bedeuten,
Z die gleichen Bedeutungsmöglichkeiten wie $R_1$ hat, wobei Z jedoch von der Gruppe $—R_1—Y^{\ominus}\ M^{\oplus}$ verschieden ist,
$Y^{\ominus}$ $SO_3^{\ominus}$, $SO_2^{\ominus}$ oder $COO^{\ominus}$ und
$M^{\oplus}$ ein Kation bedeuten, dadurch gekennzeichnet, dass man eine Phosphoniumverbindung der Formel

$$(R)_3\overset{\oplus}{P}—CH_2—X—CH_2—\overset{\oplus}{P}(R)_3 \quad Q^{\ominus} \quad Q^{\ominus}$$

worin X die vorstehend angegebene Bedeutung hat, R Alkyl mit 1 bis 6 C-Atomen, Cycloalkyl mit 5 bis 7 C-Atomen, $—NX_1X_2$, worin $X_1$ und $X_2$ unabhängig voneinander für Alkyl mit 1 bis 4 C-Atomen oder zusammen mit dem N-Atom für einen 5- oder 6-gliedrigen gesättigten heterocyclischen Ring stehen; oder unsubstituiertes oder durch Alkyl mit 1—6 C-Atomen, Alkoxy mit 1—6 C-Atomen, Chlor, Fluor,

Brom, Jod, Cycloalkyl mit 5—7 Ringgliedern, Phenyl, Phenoxy, Naphthoxy oder durch die Gruppe $—NX_1X_2$, worin $X_1$ und $X_2$ wie vorstehend definiert sind, substituiertes Phenyl und $Q^\ominus$ ein Anion bedeuten, in Gegenwart einer stark basischen Verbindung in einem polaren Lösungsmittel mit einem Aldehyd der Formel

$$M^\oplus\ Y^\ominus—R_1—CHO$$

worin $R_1$, $Y^\ominus$ und $M^\oplus$ wie oben definiert sind, bei Temperaturen zwischen 20 und 150°C umsetzt und das erhaltene Reaktionsprodukt der Formel

$$(R)_3\overset{\oplus}{P}—CH_2—X—CH{=}CH—R_1—Y^\ominus$$

nach dessen Isolierung oder direkt in der Reaktionsmischung mit einem Aldehyd der Formel Z—CHO bei Temperaturen von 100 bis 200°C in Gegenwart einer stark basischen Verbindung in einem bei über 100°C siedenden polaren Lösungsmittel umsetzt.

11. Verfahren gemäss Anspruch 10 zur Herstellung von unsymmetrischen optischen Aufhellern der Formel

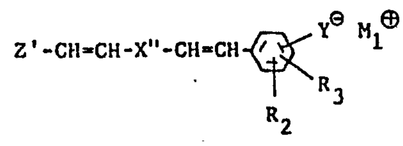

worin $M_1^\oplus$ ein Wasserstoff-, Alkalimetall-, Erdalkalimetall-, Ammonium- oder Aminion,
$Y^\ominus$ $—SO_3^\ominus$, $—SO_2^\ominus$ oder $—COO^\ominus$
$R_2$ Wasserstoff, Chlor, Brom, Fluor, Alkyl oder Alkoxy mit jeweils 1 bis 4 C-Atomen oder die Gruppe $Y^\ominus M_1^\oplus$,
$R_3$ Wasserstoff, Chlor, Brom, Fluor oder Alkyl oder Alkoxy mit jeweils 1 bis 4 C-Atomen,
X'' 1,4-Phenylen, 4,4'-Biphenylen oder 2,6-Naphthylen und
Z' eine Gruppe der Formel

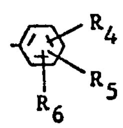 oder 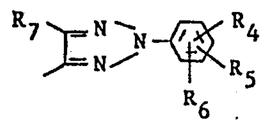 .

worin $R_4$ Wasserstoff, Chlor, Brom, Fluor, Alkyl oder Alkoxy mit jeweils 1 bis 4 C-Atomen, Cyano oder zusammen mit $R_5$ in ortho-Stellung zueinander den Rest $—O—CH_2—O—$ oder $—O—CH_2—O—CH_2—$,
$R_5$ Wasserstoff, Chlor, Brom, Fluor, Alkyl oder Alkoxy mit jeweils 1 bis 4 C-Atomen oder zusammen mit $R_4$ in ortho-Stellung zueinander den Rest $—O—CH_2—O—$ oder $—O—CH_2—O—CH_2—$,
$R_6$ Wasserstoff, Chlor, Brom, Fluor oder Alkyl mit 1 bis 4 C-Atomen und
$R_7$ Wasserstoff, Alkyl mit 1 bis 4 C-Atomen, Chlor oder Phenyl darstellen, durch Umsetzung einer Phosphoniumverbindung der Formel

$$\underset{Q^\ominus}{(R')_3\ \overset{\oplus}{P}}—CH_2—X''—CH_2—\underset{Q^\ominus}{\overset{\oplus}{P}\ (R')_3}$$

worin X'' die vorstehende Bedeutung hat,
$Q^\ominus$ für ein Anion und
R' für Alkyl mit 1 bis 4 C-Atomen, Cyclohexyl oder Phenyl steht, mit einem Aldehyd der Formel

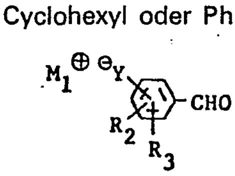

worin $R_2$, $R_3$, $Y^\ominus$ und $M_1^\ominus$ wie oben definiert sind, Isolierung des erhaltenen Zwischenproduktes der Formel

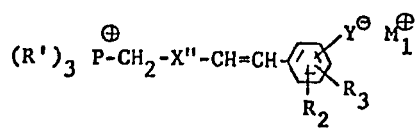

und Umsetzung des letzteren mit einem Aldehyd der Formel Z'—CHO.

12. Verfahren gemäss Anspruch 11 zur Herstellung von unsymmetrischen optischen Aufhellern der Formel

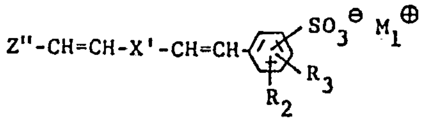

worin $M_1^{\oplus}$, $R_2$ und $R_3$ wie in Anspruch 11 definiert sind,
X' 1,4-Phenylen oder 4,4'-Biphenylen und
Z'' eine Gruppe der Formel

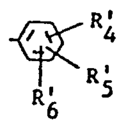 oder 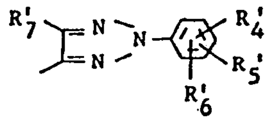

bedeuten,
worin $R_4'$ Wasserstoff, Chlor, Methyl, Methoxy oder Aethoxy oder gemeinsam mit $R_5'$ in ortho-Stellung den Rest $-O-CH_2-O-$ oder $-O-CH_2-O-CH_2-$,
$R_5'$ Wasserstoff, Chlor, Methyl, Methoxy oder Aethoxy oder gemeinsam mit $R_4'$ in ortho-Stellung den Rest $-O-CH_2-O-$ oder $-O-CH_2-O-CH_2-$,
$R_6'$ Wasserstoff, Chlor oder Methyl und
$R_7'$ Wasserstoff, Methyl oder Aethyl darstellen, durch Umsetzung einer Phosphoniumverbindung der Formel

$$(R')_3\overset{\oplus}{P}-CH_2-X'-CH_2-\overset{\oplus}{P}(R')_3$$
$$Q^{\ominus} \qquad\qquad Q^{\ominus}$$

worin R' und $Q^{\ominus}$ wie in Anspruch 11 und X' wie oben definiert sind, mit einem Aldehyd der Formel

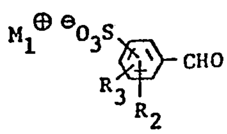

worin $M_1^{\oplus}$, $R_2$ und $R_3$ wie oben definiert sind, und Umsetzung des erhaltenen Zwischenproduktes mit einem Aldehyd der Formel Z''—CHO.

13. Verfahren gemäss Anspruch 12 zur Herstellung von unsymmetrischen optischen Aufhellern der Formel

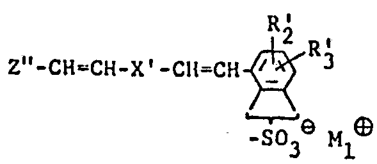

worin X', Z'' und $M_1^{\oplus}$ wie in Anspruch 12 definiert sind und
$R_2'$ und $R_3'$ unabhängig voneinander jeweils Wasserstoff, Chlor, Methyl, Methoxy oder Aethoxy bedeuten, durch Umsetzung einer Phosphoniumverbindung der Formel

$$(R')_3\overset{\oplus}{P}-CH_2-X'-CH_2-\overset{\oplus}{P}(R')_3$$
$$Q^{\ominus} \qquad\qquad Q^{\ominus}$$

worin die allgemeinen Symbole wie in Anspruch 12 definiert sind, mit einem Aldehyd der Formel

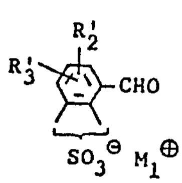

worin $R_2'$, $R_3'$ und $M_1^{\oplus}$ wie oben definiert sind, und Umsetzung des erhaltenen Zwischenproduktes mit einem Aldehyd der Formel Z''—CHO.

14. Verfahren gemäss Anspruch 13 zur Herstellung von unsymmetrischen optischen Aufhellern der Formel

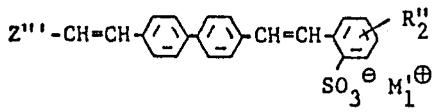

worin $R_2''$ Wasserstoff oder Chlor,
$M_1'^{\oplus}$ ein Wasserstoff-, Natrium-, Kalium- oder Ammoniumion und
$Z'''$ einen Rest der Formel

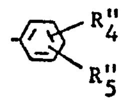 oder 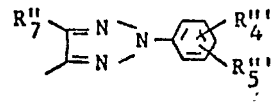

bedeuten, worin $R_4''$ Wasserstoff, Chlor, Methyl, Methoxy oder zusammen mit $R_5''$ in ortho-Stellung die Gruppe der Formel $-O-CH_2-O-CH_2-$,
$R_5''$ Wasserstoff, Chlor, Methyl oder zusammen mit $R_4''$ in ortho-Stellung die Gruppe der Formel $-O-CH_2-O-CH_2-$,
$R_4'''$ Wasserstoff, Chlor, Methyl, Methoxy oder Aethoxy,
$R_5'''$ Wasserstoff, Chlor oder Methyl und
$R_7''$ Wasserstoff oder Methyl darstellen, durch Umsetzung einer, Phosphoniumverbindung der Formel

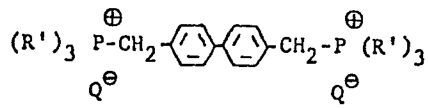

worin die allgemeinen Symbole wie in Anspruch 13 definiert sind, mit einem Aldehyd der Formel

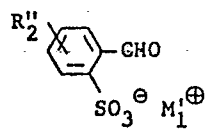

worin $R_2''$ und $M_1'^{\oplus}$ wie oben definiert sind, und Umsetzung des erhaltenen Zwischenproduktes mit einem Aldehyd der Formel $Z'''-CHO$.

15. Verfahren gemäss Anspruch 13 zur Herstellung von unsymmetrischen optischen Aufhellern der Formel

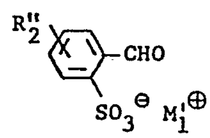

worin $Z'''$, $R_2''$ und $M_1'^{\oplus}$ wie in Anspruch 14 definiert sind, durch Umsetzung einer Phosphoniumverbindung der Formel

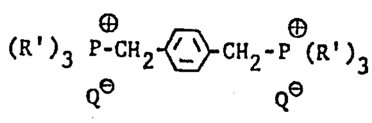

worin R′ und $Q^{\ominus}$ wie in Anspruch 14 definiert sind, mit einem Aldehyd der Formel

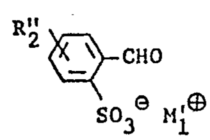

und Umsetzung des erhaltenen Zwischenproduktes mit einem Aldehyd der Formel $Z'''-CHO$.

16. Verfahren gemäss Anspruch 10, dadurch gekennzeichnet, dass man die erste Stufe in Gegenwart eines Natrium- oder Kaliumalkoholats in einem niedrigen primären aliphatischen Alkohol als Lösungsmittel bei Temperaturen zwischen 20 und 100°C und die zweite Stufe nach Isolierung des Zwischenproduktes in Gegenwart eines Natrium- oder Kaliumalkoholats, eines Natrium- oder Kaliumhydroxides oder von metallischem Natrium oder Kalium in einem bei mindestens 100°C siedenden Lösungsmittel, vorzugsweise in einem höheren Alkohol wie Aethylenglykol bei Temperaturen zwischen 100 und 200°C durchführt.

**Claims**

1. A phosphonium compound of the formula

$$(R)_3 \overset{\oplus}{P}—CH_2—X—CH=CH—R_1—Y^{\ominus}$$

in which R is alkyl having 1 to 6 C atoms, cycloalkyl having 5 to 7 C atoms, the group $—NX_1X_2$, in which $X_1$ and $X_2$ independently of each other are alkyl having 1 to 4 C atoms or together with the N atom are a 5-membered or 6-membered saturated heterocyclic ring, or is phenyl which is unsubstituted or substituted by alkyl having 1 to 6 C atoms, alkoxy having 1 to 6 C atoms, chlorine, fluorine, bromine, iodine, cycloalkyl having 5 to 7 ring members, phenyl, phenoxy, naphthoxy or by the $—NX_1X_2$ group, wherein $X_1$ and $X_2$ are as defined above; $R_1$ is a phenyl, naphthyl, furan, thiophene, oxazole, isoxazole, pyrazole, imidazole triazole or oxadiazole radical which in addition to the group $Y^{\ominus}$ can carry further non-chromophoric substituents, it being possible for two substituents in the ortho-position also to form the member necessary to complete a fused benzo or naphtho radical which is unsubstituted or substituted by non-chromophoric groups, X is phenylene, 4,4'-biphenylene or 1,4- or 2,6-naphthylene and $Y^{\ominus}$ is $SO_3^{\ominus}$, $SO_2^{\ominus}$ or $COO^{\ominus}$.

2. A phosphonium compound according to claim 1, of the formula

$$(R')_3 \overset{\oplus}{P}—CH_2—X''—CH=CH—R_1'—Y^{\ominus}$$

in which $R_1'$ is a phenyl, naphthyl, furan, thiophene, oxazole, isoxazole, pyrazole, imidazole, triazole or oxadiazole radical which in addition to the group $Y^{\ominus}$ can carry one or two further non-chromophoric substituents, it being possible for two adjacent substituents also to form the member necessary to complete a fused benzo or naphtho radical,
R' is alkyl having 1 to 4 C atoms, cyclohexyl or phenyl,
$Y^{\ominus}$ is $SO_3^{\ominus}$, $SO_2^{\ominus}$ or $COO^{\ominus}$ and
X'' is 1,4-phenylene, 2,6-naphthylene or 4,4'-biphenylene.

3. A phosphonium compound according to claim 2, of the formula

$$(R')_3 \overset{\oplus}{P}—CH_2—X'—CH=CH—R_1''—Y_1^{\ominus}$$

in which $R_1''$ is a phenyl or furan radical which, in addition to the group $Y_1^{\ominus}$, can carry one or two further substituents from the group comprising alkyl having 1 to 4 C atoms, alkoxy having 1 to 4 C atoms, cyclohexyl, alkenyl having 3 to 4 C atoms, phenyl, sulfo, fluorine chlorine, bromine or carbalkoxy having 2 to 5 C atoms,
R' is alkyl having 1 to 4 C atoms, cyclohexyl or phenyl,
$Y_1^{\ominus}$ is $SO_3^{\ominus}$ or $COO^{\ominus}$ and
X' is 1,4-phenylene or 4,4'-biphenylene.

4. A phosphonium compound according to claim 1 of the formula

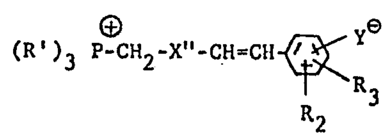

in which $Y^{\ominus}$ is $—SO_3^{\ominus}$, $—SO_2^{\ominus}$ or $—COO^{\ominus}$,
$R_2$ is hydrogen, chlorine, bromine, fluorine, alkyl or alkoxy each having 1 to 4 C atoms or a further group $Y^{\ominus}$,
$R_3$ is hydrogen, chlorine, bromine, fluorine or alkyl or alkoxy each having 1 to 4 C atoms,
X'' is 1,4-phenylene, 4,4'-biphenylene or 2,6-naphthylene and R' is alkyl having 1 to 4 C atoms, cyclohexyl or phenyl.

5. A phosphonium compound according to claim 4 of the formula

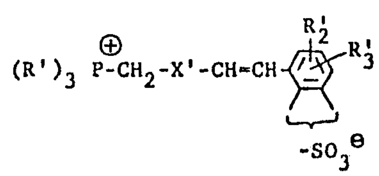

in which R' is alkyl having 1 to 4 C atoms, cyclohexyl or phenyl, each of $R_2'$ and $R_3'$ independently of the other is hydrogen, chlorine, methyl, methoxy or ethoxy and X' is 1,4-phenylene or 4,4'-biphenylene.

6. A process for the preparation of a phosphonium compound of the formula as indicated in claim 1, which process comprises reacting a phosphonium compound of the formula

$$(R)_3 \overset{\oplus}{P}—CH_2—X—CH_2—\overset{\oplus}{P} (R)_3$$
$$Q^{\ominus} \qquad Q^{\ominus}$$

wherein R and X are as defined in claim 1 and $Q^{\ominus}$ is an anion, with an aldehyde of the formula

$$M^{\oplus} Y^{\ominus}—R_1—CHO$$

wherein $R_1$ and $Y^{\ominus}$ are as defined in claim 1 and $M^{\oplus}$ is a cation, said process being conducted in the presence of a strong basic compound and a polar solvent.

7. A process according to claim 6, for the preparation of a phosphonium compound of the formula

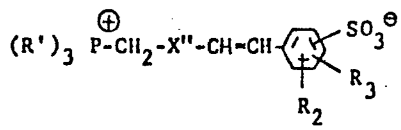

in which $R_2$ is hydrogen, chlorine, bromine, fluorine, alkyl or alkoxy each having 1 to 4 C atoms or a sulfo group, $R_3$ is hydrogen, chlorine, bromine, fluorine or alkyl or alkoxy each having 1 to 4 C atoms, X″ is 1,4-phenylene, 4,4′-biphenylene or 2,6-naphthylene and R′ is alkyl having 1 to 4 C atoms, cyclohexyl or phenyl, which process comprises reacting a phosphonium compound of the formula

$$(R')_3 \overset{\oplus}{P}—CH_2—X''—CH_2—\overset{\oplus}{P} (R')_3$$
$$Q_1^{\ominus} \qquad Q_1^{\ominus}$$

in which R′ and X″ are as defined above and $Q_1^{\oplus}$ is a chloride or bromide ion, with an aldehyde of the formula

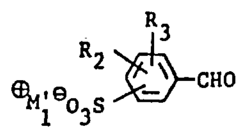

in which $R_2$ and $R_3$ are as defined above and $M_1'^{\oplus}$ is a hydrogen, sodium, potassium or ammonium ion.

8. A process according to claim 6, which comprises carrying out the reaction in the presence of a sodium alcoholate or potassium alcoholate in the temperature range from 20° to 150°C.

9. A process according to claim 6, wherein the polar solvent is an alcohol.

10. A process for the preparation of an asymmetrical fluorescent whitening agent of the formula

$$Z—CH=CH—X—CH=CH—R_1—Y^{\ominus}M^{\oplus}$$

in which $R_1$ is a phenyl, naphthyl, furan, thiophene, oxazole, isoxazole, pyrazole, imidazole, triazole or oxadiazole radical which in addition to the group $Y^{\ominus}$ can carry further non-chromophoric substituents, it being possible for two substituents in the ortho position also to form the member necessary to complete a fused benzo or naphtho radical which is unsubstituted or substituted by non-chromophoric groups,

X is phenylene, 4,4′-biphenylene or 1,4- or 2,6-naphthylene,

Z has the same meanings as $R_1$ but differs from the group $—R_1—Y^{\ominus}M^{\oplus}$,

$Y^{\ominus}$ is $SO_3^{\ominus}$, $SO_2^{\ominus}$ or $COO^{\ominus}$ and

$M^{\oplus}$ is a cation, which process comprises reacting a phosphonium compound of the formula

$$(R)_3 \overset{\oplus}{P}—CH_2—X—CH_2—\overset{\oplus}{P} (R)_3$$
$$Q^{\ominus} \qquad Q^{\ominus}$$

in which X is as defined above, R is alkyl having 1 to 6 C atoms, cycloalkyl having 5 to 7 C atoms, $—NX_1X_2$, in which each of $X_1$ and $X_2$ independently of the other is alkyl having 1 to 4 C atoms or both together with the N atom are a 5-membered or 6-membered saturated heterocyclic ring, or is phenyl which is unsubstituted or substituted by alkyl having 1 to 6 C atoms, alkoxy having 1 to 6 C atoms, chlorine, fluorine, bromine, iodine, cycloalkyl having 5 to 7 ring members, phenyl, phenoxy, naphthoxy or by the $—NX_1X_2$ group, wherein $X_1$ and $X_2$ are as defined above and

$Q^{\ominus}$ is an anion, in the presence of a strongly basic compound in a polar solvent, with an aldehyde of the formula

$$M^{\oplus}Y^{\ominus}—R_1—CHO$$

in which $R_1$, $Y^{\ominus}$ and $M^{\oplus}$ are as defined above, in the temperature range from 20° to 150°C, and reacting the resultant reaction product of the formula

$$(R)_3\overset{\oplus}{P}—CH_2—X—CH{=}CH—R_1—Y^{\ominus}$$

after it has been isolated or direct in the reaction mixture, with an aldehyde of the formula Z—CHO in the temperature range from 100° to 200°C in the presence of a strongly basic compound in a polar solvent which boils above 100°C.

11. A process according to claim 10, for the preparation of an asymmetrical fluorescent brightening agent of the formula

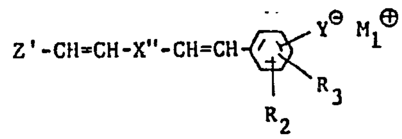

in which $M_1^{\oplus}$ is a hydrogen, alkali metal, alkaline earth metal, ammonium or amine ion,
$Y^{\ominus}$ is $—SO_3^{\ominus}$, $—SO_2^{\ominus}$ or $—COO^{\ominus}$,
$R_2$ is hydrogen, chlorine, bromine, fluorine, alkyl or alkoxy each having 1 to 4 C atoms or the group $Y^{\ominus}M_1^{\oplus}$,
$R_3$ is hydrogen, chlorine, bromine, fluorine or alkyl or alkoxy each having 1 to 4 C atoms,
X'' is 1,4-phenylene, 4,4'-biphenylene or 2,6-naphthylene and Z' is a group of the formula

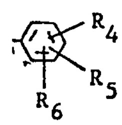 or 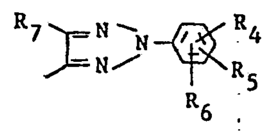

in which $R_4$ is hydrogen, chlorine, bromine, fluorine, alkyl or alkoxy each having 1 to 4 C atoms or cyano, or together with $R_5$ in the ortho-position relative to each other is the radical $—O—CH_2—O—$ or $—O—CH_2—O—CH_2—$, $R_5$ is hydrogen, chlorine, bromine, fluorine or alkyl or alkoxy each having 1 to 4 C atoms or together with $R_4$ in the ortho-position relative to one another is the radical $—O—CH_2—O—$ or $—O—CH_2—O—CH_2—$,
$R_6$ is hydrogen, chlorine, bromine, fluorine or alkyl having 1 to 4 C atoms and
$R_7$ is hydrogen, alkyl having 1 to 4 C atoms, chlorine or phenyl, which process comprises reacting a phosphonium compound of the formula

$$\underset{Q^{\ominus}}{(R')_3\overset{\oplus}{P}}—CH_2—X''—CH_2—\underset{Q^{\ominus}}{\overset{\oplus}{P}(R')_3}$$

in which X'' is as defined above, $Q^{\ominus}$ is an anion and R' is alkyl having 1 to 4 C atoms, cyclohexyl or phenyl, with an aldehyde of the formula

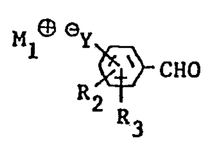

in which $R_2$, $R_3$, $Y^{\ominus}$ and $M_1^{\oplus}$ are as defined above, isolating the resultant intermediate of the formula

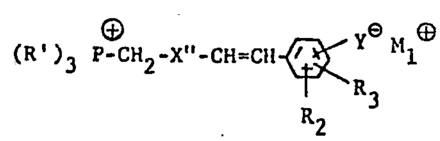

and reacting this latter with an aldehyde of the formula Z'—CHO.

12. A process according to claim 11, for the preparation of an asymmetrical fluorescent whitening agent of the formula

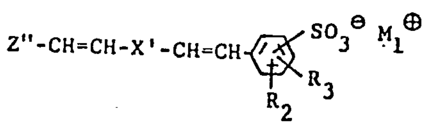

in which $M_1^{\oplus}$, $R_2$ and $R_3$ are as defined in claim 11.

X' is 1,4-phenylene or 4,4'-biphenylene and
Z'' is a group of the formula

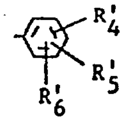 or 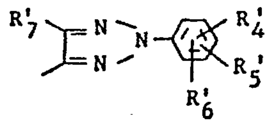

in which $R_4'$ is hydrogen, chlorine, methyl, methoxy or ethoxy together with $R_5'$ in the ortho-positon is the radical $—O—CH_2—O—$ or $—O—CH_2—O—CH_2—$,
$R_5'$ is hydrogen, chlorine, methyl, methoxy or ethoxy, or together with $R_4'$ in the ortho-position is the radical $—O—CH_2—O—$ or $—O—CH_2—O—CH_2—$,
$R_6'$ is hydrogen, chlorine or methyl and
$R_7'$ is hydrogen, methyl or ethyl, which process comprises reacting a phosphonium compound of the formula

$$(R')_3\overset{\oplus}{P}—CH_2—X'—CH_2—\overset{\oplus}{P}(R')_3 \quad Q^{\ominus} \quad Q^{\ominus}$$

in which R' and $Q^{\ominus}$ are as defined in claim 11 and X' is as defined above, with an aldehyde of the formula

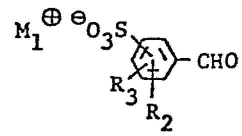

in which $M_1^{\oplus}$, $R_2$ and $R_3$ are as defined above, and reacting the resulting intermediate with an aldehyde of the formula Z''—CHO.

13. A process according to claim 12, for the preparation of an asymmetrical fluorescent whitening agent of the formula

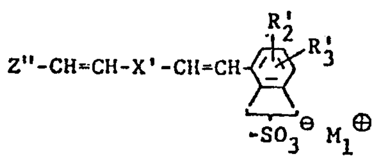

in which X', Z'' and $M_1^{\oplus}$ are as defined in claim 12 and each of $R_2'$ and $R_3'$ independently of the other is hydrogen, chlorine, methyl, methoxy or ethoxy, which process comprises reacting a phosphonium compound of the formula

$$(R')_3\overset{\oplus}{P}—CH_2—X'—CH_2—\overset{\oplus}{P}(R')_3 \quad Q^{\ominus} \quad Q^{\ominus}$$

in which the general symbols are as defined in claim 12, with an aldehyde of the formula

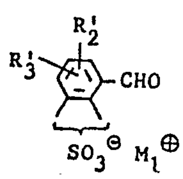

in which $R_2'$, $R_3'$ and $M_1^{\oplus}$ are as defined above, and reacting the resultant intermediate with an aldehyde of the formula Z''—CHO.

14. A process according to claim 13, for the preparation of an asymmetrical fluorescent brightening agent of the formula

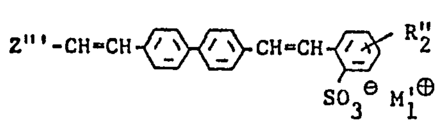

in which $R_2''$ is hydrogen or chlorine, $M_1'^{\oplus}$ is a hydrogen, sodium, potassium or ammonium ion and Z''' is a radical of the formula

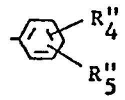 or 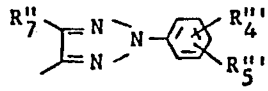

in which $R_4''$ is hydrogen, chlorine, methyl or methoxy or together with $R_5''$ in the ortho-position is the group of the formula $-O-CH_2-O-CH_2-$,
$R_5''$ is hydrogen, chlorine or methyl together with $R_4''$ in the ortho-position is the group of the formula $-O-CH_2-O-CH_2-$,
$R_4'''$ is hydrogen, chlorine, methyl, methoxy or ethoxy,
$R_5'''$ is hydrogen, chlorine or methyl and
$R_7''$ is hydrogen or methyl, which process comprises reacting a phosphonium compound of the formula

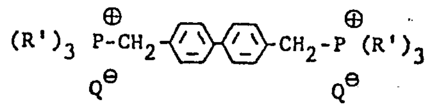

in which the general symbols are as defined in claim 13, with an aldehyde of the formula

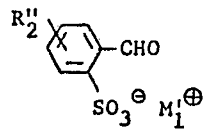

in which $R_2''$ and $M_1'^{\oplus}$ are as defined above, and reacting the resultant intermediate with an aldehyde of the formula $Z'''-CHO$.

15. A process according to claim 13, for the preparation of an asymmetrical fluorescent brightening agent of the formula

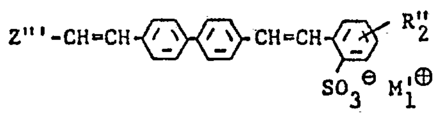

in which $Z'''$, $R_2''$ and $M_1'^{\oplus}$ are as defined in claim 14, which process comprises reacting a phosphonium compound of the formula

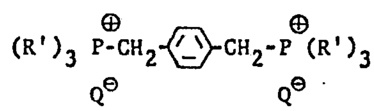

in which $R'$ and $Q^{\ominus}$ are as defined in claim 14, with an aldehyde of the formula

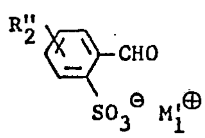 .

and reacting the resultant intermediate with an aldehyde of the formula $Z'''-CHO$.

16. A process according to claim 10, which comprises carrying out the first stage in the presence of a sodium alcoholate or potassium alcoholate in a lower primary aliphatic alcohol as solvent, in the temperature range from 20° to 100°C, and carrying out the second stage, after isolation of the intermediate, in the presence of a sodium alcoholate, potassium alcoholate, sodium hydroxide, potassium hydroxide, metallic sodium or metallic potassium, in a solvent which boils not lower than 100°C, preferably in a higher alcohol such as ethylene glycol in the temperature range from 100° to 200°C.

**Revendications**

1. Composés de phosphonium der formule:

$$(R)_3 \overset{\oplus}{P}-CH_2-X-CH=CH-R_1-Y^{\ominus}$$

dans laquelle R est un reste alkyle avec 1 à 6 atomes de carbone, cycloalkyle avec 5 à 7 atomes de carbone, $-NX_1X_2$, où $X_1$ et $X_2$, indépendamment l'un de l'autre, sont des radicaux alkyle avec 1 à 4 atomes de carbone, ou ensemble avec l'atome N forment un noyau hétérocyclique saturé à 5 ou 6

chaînons; ou bien R est un reste phényle non substitué ou substitué par des radicaux alkyle avec 1 à 6 atomes de carbone, alcoxy avec 1 à 6 atomes de carbone, chloro, fluoro, bromo, iodo, cycloalkyle avec 5 à 7 chaînons, phényle, phénoxy, naphthoxy, ou bien par le group $—NX_1X_2$, où $X_1$ et $X_2$ sont définis comme précédemment;

$R_1$ est un reste phényle, naphtyle, furanne, thiophène, oxazole, isoxazole, pyrazole, imidazole, triazole ou oxadiazole présentant, en plus du groupe $Y^{\ominus}$, éventuellement encore des substituants non-chromophores, deux substituants, en position ortho-, pouvant former également le complément à un reste benzo ou napho non-substitué ou substitué par un group non-chromophore;

X est un reste phénylène, 4,4'-biphénylène ou 1,4- ou 2,6-naphthylène, et

$Y^{\ominus}$ est $SO_3^{\ominus}$, $SO_2^{\ominus}$ ou $COO^{\ominus}$.

2. Composés de phosphonium selon la revendication 1 ayant la formule:

$$(R')_3\ \overset{\oplus}{P}—CH_2—X''—CH=CH—R_1'—Y^{\ominus}$$

dans laquelle $R_1'$ est un reste phényle, naphtyle, furanne, thiophène, oxazole, isoxazole, pyrazole, imidazole, triazole ou oxadiazole portant, en plus du groupe $Y^{\ominus}$, éventuellement un ou deux autres substituants non-chromophores, deux substituants voisins pouvant former également le complément à un reste benzo ou naphto, fixé par condensation;

R' est un reste alkyle avec 1 à 4 atomes de carbone, cyclohexyle ou phényle;

$Y^{\ominus}$ est $SO_3^{\ominus}$, $SO_2^{\ominus}$ ou $COO^{\ominus}$, et

X'' est un reste 1,4-phénylène, 2,6-naphtylène ou 4,4'-biphénylène.

3. Composés de phosphonium selon la revendication 2, ayant la formule:

$$(R')_3\ \overset{\oplus}{P}—CH_2—X'—CH=CH—R_1''—Y^{\ominus}$$

dans laquelle $R_1''$ est un reste phényle ou furanne portant, en plus du groupe $Y^{\ominus}$ éventuellement un ou deux autres substituants constitués par des groupes alkyle avec 1 à 4 atomes de carbone, alcoxy avec 1 à 4 atomes de carbone, cyclohexyle, alcényle avec 3 ou 4 atomes de carbone, phényle, sulfo, fluoro, chloro, bromo ou carbalcoxy avec 2 à 5 atomes de carbone;

R' est un reste alkyle avec 1 à 4 atomes de carbone, cyclohexyle ou phényle;

$Y_1^{\ominus}$ est $SO_3^{\ominus}$ ou $COO^{\ominus}$, et

X' est un reste 1,4-phénylène ou 4,4'-biphénylène.

4. Composés de phosphonium selon la revendication 1 ayant la formule:

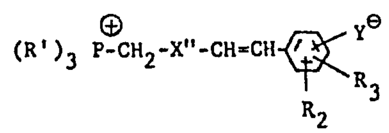

dans laquelle $Y^{\ominus}$ est $—SO_3^{\ominus}$, $—SO_2^{\ominus}$ ou $—COO^{\ominus}$;

$R_2$ est de l'hydrogène, du chlore, du brome, du fluor, un reste alkyle ou alcoxy ayant chacun 1 à 4 atomes de carbone, ou un autre groupe $Y^{\ominus}$;

$R_3$ est de l'hydrogène, du chlore, du brome, du fluor, un reste alkyle ou alcoxy ayant chacun 1 à 4 atomes de carbone;

X'' est un reste 1,4-phénylène, 4,4'-biphénylène ou 2,6-naphtylène, et

R' est une reste alkyle avec 1 à 4 atomes de carbone, cyclohexyle ou phényle.

5. Composés de phosphonium selon la revendication 4, ayant la formule:

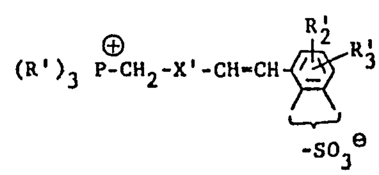

dans laquelle R' est un reste alkyle avec 1 à 4 atomes de carbone, cyclohexyle ou phényle;

$R_2'$ et $R_3'$, indépendamment l'un de l'autre, sont chacun de l'hydrogène, du chlore, un reste méthyle, méthoxy ou éthoxy, et

X' est un reste 1,4-phénylène ou 4,4'-biphénylène.

6. Procédé pour la préparation de composés de phosphonium de formule donnée dans la revendication 1, caractérisé par le fait qu'on fait réagir un composé de phosphonium de formule:

$$(R)_3\ \overset{\oplus}{P}—CH_2—X—CH_2—\overset{\oplus}{P}\ (R)_3 \qquad Q^{\ominus} \quad Q^{\ominus}$$

dans laquelle R et X sont définis comme dans la revendication 1, et $Q^{\ominus}$ est un anion, en présence d'un

composé fortement basique et d'un solvant polaire, avec un aldéhyde de formule:

$$M^{\oplus}Y^{\ominus}—R_1—CHO,$$

dans laquelle $R_1$ et $Y^{\ominus}$ sont définis comme dans la revendication 1, et $M^{\oplus}$ est un cation.

7. Procédé selon la revendication 6 pour la préparation de composés de phosphonium de formule:

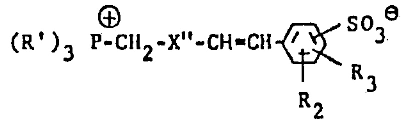

dans laquelle $R_2$ est de l'hydrogène, du chlore, du brome, du fluor, un reste alkyle ou alcoxy avec chacun 1 à 4 atomes de carbone, ou bien un groupe sulfo;
$R_3$ est de l'hydrogène, du chlore, du brome, du fluor, un reste alkyle ou alcoxy avec chacun 1 à 4 atomes de carbone;
$X''$ est un reste 1,4-phénylène, 4,4'-biphénylène ou 2,6-naphthylène, et
$R'$ est un reste alkyle avec 1 à 4 atomes de carbone, cyclohexyle ou phényle, en faisant réagir un composé de phosphonium de formule:

$$(R')_3\overset{\oplus}{P}—CH_2—X''—CH_2—\overset{\oplus}{P}(R')_3 \qquad Q_1^{\ominus} \qquad Q_1^{\ominus}$$

dans laquelle $R'$ et $X''$ ont les significations données précédemment, et $Q_1^{\ominus}$ est un ion chlorure ou bromure, avec un aldéhyde de formule:

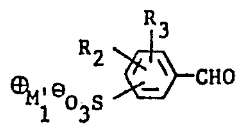

dans laquelle $R_2$ et $R_3$ ont les significations données précédemment, et $M_1'^{\oplus}$ est un ion hydrogène, sodium potassium ou ammonium.

8. Procédé selon la revendication 6, caractérisé par le fait qu'on effectue la réaction en présence d'un alcoolate de sodium ou de potassium à des températures comprises entre 20° et 150°C.

9. Procédé selon la revendication 6, caractérisé par la fait qu'on utilise comme solvant polaire un alcool.

10. Procédé pour la préparation d'azurants optiques asymétriques de formule:

$$Z—CH=CH—X—CH=CH—R_1—Y^{\ominus}M^{\oplus}$$

dans laquelle $R_1$ est un reste phényle, naphtyle, furanne, thiophène, oxazole, isoxazole, pyrazole, imidazole, triazole ou oxadiazole présentant, en plus du groupe $Y^{\ominus}$, éventuellement d'autres substituants non-chromophores, deux substituants, en position ortho-, pouvant former également le complément à un reste benzo ou naphto non substitué ou substitué par un groupe non-chromophore, et
X est un reste phénylène, 4,4'-biphénylène ou 1,4- ou 2,6-naphtylène;
Z a la même possibilité de signification que $R_1$, mais Z étant différent du groupe $—R_1—Y^{\ominus}M^{\oplus}$,
$Y^{\ominus}$ est $SO_3^{\ominus}$, $SO_2^{\ominus}$ ou $COO^{\ominus}$, et
$M^{\oplus}$ est un cation, caractérisé par le fait qu'on fait réagir un composé de phosphonium de formule:

$$(R)_3\overset{\oplus}{P}—CH_2—X—CH_2—\overset{\oplus}{P}(R)_3 \qquad Q^{\ominus} \qquad Q^{\ominus}$$

dans laquelle X a les significations données précédemment;
R est un reste alkyle avec 1 à 6 atomes de carbone, cycloalkyle avec 5 à 7 atomes de carbone, $—NX_1X_2$, où $X_1$ et $X_2$, indépendamment l'un de l'autre, sont chacun un radical alkyle avec 1 à 4 atomes de carbone, ou, en commun avec l'atome d'azote, forment un noyau hétérocyclique saturé à 5 ou 6 chaînons; ou bien R est un reste phényle non substitué ou substitué par des radicaux alkyle avec 1 à 6 atomes de carbone, alcoxy avec 1 à 6 atomes de carbone, chloro, fluoro, bromo, iodo, cycloalkyle avec 5 à 7 chaînons, phényle, phénoxy, naphtoxy ou bien par le groupe $—NX_1X_2$, où $X_1$ et $X_2$ sont définis comme précédemment, et
$Q^{\ominus}$ est un anion, en présence d'un composé fortement basique, dans un solvant polaire, avec un aldéhyde de formule:

$$M^{\oplus}Y^{\ominus}—R_1—CHO$$

où $R_1$, $Y^{\ominus}$ et $M^{\oplus}$ sont définis ci-dessus, à des température comprises entre 20° e 150°C, puis en faisant réagir le produit de réaction obtenu:

$$(R)_3\overset{\oplus}{P}—CH_2—X—CH{=}CH—R_1—Y^{\ominus}$$

après son isolement, ou directement dans le mélange réactionnel, avec un aldéhyde de formule Z—CHO, à des températures de 100 à 200°C, en présence d'un composé fortement basique, dans un solvant polaire bouillant au-dessus de 100°C.

11. Procédé selon la revendication 10 pour la préparation d'azurants optiques asymétriques de formule:

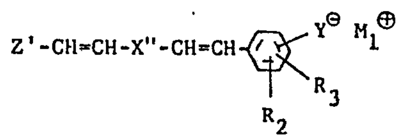

dans laquelle $M_1^{\oplus}$ est un ion hydrogène, métal alcalin, métal alcalinoterreux, ammonium ou amine;
$Y^{\ominus}$ est $—SO_3^{\ominus}$, $—SO_2^{\ominus}$ ou $—COO^{\ominus}$;
$R_2$ est de l'hydrogène, du chlore, du brome, du fluor, un reste alkyle ou alcoxy avec chacun 1 à 4 atomes de carbone ou le groupe $Y^{\ominus}$ $M_1^{\oplus}$;
$R_3$ est de l'hydrogène, du chlore, du brome, du fluor ou un reste alkyle ou alcoxy avec chacun 1 à 4 atomes de carbone;
X'' est un reste 1,4-phénylène, 4,4'-biphénylène ou 2,6-naphtylène, et
Z' est un groupe de formules:

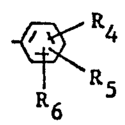 ou 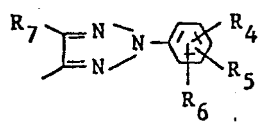

dans lesquelles $R_4$ est de l'hydrogène, du chlore, du brome, du fluor, un reste alkyle ou alcoxy avec chacun 1 à 4 atomes de carbone, cyano, ou, ensemble avec $R_5$ en position ortho- l'un par rapport à l'autre, le reste $—O—CH_2—O—$ ou $—O—CH_2—O—CH_2—$;
$R_5$ est de l'hydrogène, du chlore, du brome, du fluor, un reste alkyle ou alcoxy avec chacun 1 à 4 atomes de carbone, ou bien ensemble avec $R_4$ en position ortho- l'un par rapport à l'autre, le $—O—CH_2—O—$ ou $—O—CH_2—O—CH_2—$;
$R_6$ est de l'hydrogène, du chlore, du brome, du fluor, ou un reste alkyle avec 1 à 4 atomes de carbone, et
$R_7$ est de l'hydrogène, un reste alkyle avec 1 à 4 atomes de carbone, du chlore ou le reste phényle, en faisant réagir un composé de phosphonium de formule:

$$(R')_3\overset{\oplus}{P}—CH_2—X''—CH_2—\overset{\oplus}{P}(R')_3$$
$$Q^{\ominus} \qquad\qquad Q^{\ominus}$$

dans laquelle X'' a les significations précédentes;
$Q^{\ominus}$ est un anion, et
R' est un reste alkyle avec 1 à 4 atomes de carbone, cyclohexyle ou phényle, avec un aldéhyde de formule:

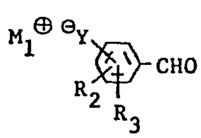

dans laquelle $R_2$, $R_3$, $Y^{\ominus}$ et $M_1^{\oplus}$ sont définis ci-dessus, en isolant le produit intermédiaire obtenu de formule:

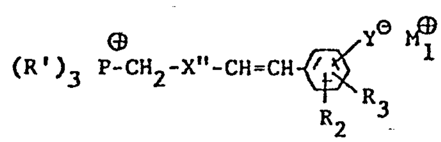

puis en faisant réagir ce dernier avec un aldéhyde de formule Z'—CHO.

12. Procédé selon la revendication 11, pour la préparation d'azurants optiques asymétriques de formule:

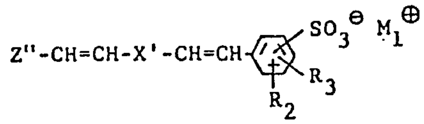

dans laquelle $M_1^{\oplus}$, $R_2$ et $R_3$ sont définis dans la revendication 11; X′ est un reste 1,4-phénylène ou 4,4′-biphénylène, et Z″ est un groupe de formules:

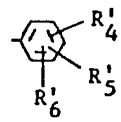

ou

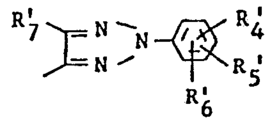

dans lesquelles $R_4'$ est de l'hydrogène, du chlore, un reste méthyle, méthoxy ou éthoxy, ou bien, un commun avec $R_5'$ en position ortho-, forme le reste $-O-CH_2-O-$ ou $-O-CH_2-O-CH_2-$; $R_5'$ est de l'hydrogène, du chlore, un reste méthyle, méthoxy ou éthoxy ou, en commun avec $R_4'$ en position ortho-, forme le reste $-O-CH_2-O-$ ou $-O-CH_2-O-CH_2-$; $R_6'$ est de l'hydrogène, du chlore ou le reste méthyle, et $R_7'$ est de l'hydrogène, un reste méthyle ou éthyle, en faisant réagir un composé de phosphonium de formule:

$$(R')_3\overset{\oplus}{P}-CH_2-X'-CH_2-\overset{\oplus}{P}(R')_3 \quad Q^{\ominus} \quad Q^{\ominus}$$

dans laquelle R′ et $Q^{\ominus}$ sont définis comme dans la revendication 11, et X′ est défini comme ci-dessus, avec un aldéhyde de formule:

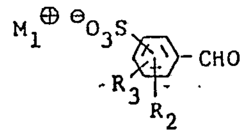

dans laquelle $M_1^{\oplus}$, $R_2$ et $R_3$ sont définis comme ci-dessus, puis en faisant réagir le produit intermédiaire obtenu avec un aldéhyde de formule Z″—CHO.

13. Procédé selon la revendication 12 pour la préparation d'azurants optiques asymétriques de formule:

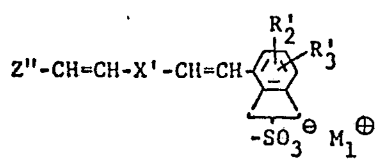

dans laquelle X′, Z″ et $M_1^{\oplus}$ sont définis comme dans la revendication 12, et $R_2'$ et $R_3'$ indépendamment l'un de l'autre sont chacun de l'hydrogène, du chlore, le reste méthyle, méthoxy ou éthoxy, en faisant réagir un composé de phosphonium de formule:

$$(R')_3\overset{\oplus}{P}-CH_2-X'-CH_2-\overset{\oplus}{P}(R')_3 \quad Q^{\ominus} \quad Q^{\ominus}$$

où les symboles généraux sont définis comme dans la revendication 12, avec un aldéhyde de formule:

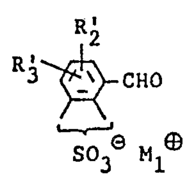

où $R_2'$, $R_3'$ et $M_1^{\oplus}$ sont définis comme ci-dessus, puis en faisant réagir le produit intermédiaire obtenu avec un aldéhyde de formule Z″—CHO.

14. Procédé selon la revendication 13 pour la préparation d'azurants optiques asymétriques de formule:

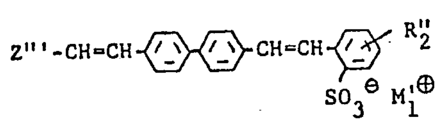

dans laquelle $R_2''$ est de l'hydrogène ou du chlore;

$M_1'^{\oplus}$ est un ion hydrogène, sodium, potassium ou ammonium, et
Z''' est un reste de formule:

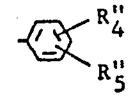

ou

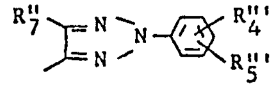

dans laquelle $R_4''$ est de l'hydrogène, du chlore, un reste méthyle, méthoxy ou, ensemble avec $R_5$ en position ortho- forme le groupe de formule —O—$CH_2$—O—$CH_2$—;
$R_5''$ est de l'hydrogène, du chlore, un reste méthyle ou, ensemble avec $R_4''$ en position ortho-, forme le groupe de formule —O—$CH_2$—O—$CH_2$—;
$R_4'''$ est de l'hydrogène, du chlore, le reste méthyle, méthoxy ou éthoxy;
$R_5'''$ est de l'hydrogène, du chlore ou le reste méthyle, et
$R_7''$ est de l'hydrogène ou le reste méthyle, en faisant réagire un composé de phosphonium de formule:

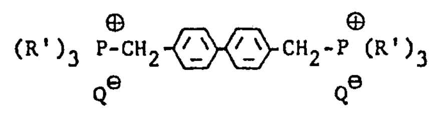

dans laquelle tous les symboles sont définis comme dans la revendication 13, avec un aldéhyde de formule:

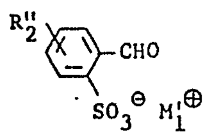

où $R_2''$ et $M_1'^{\oplus}$ sont définis ci-dessus, puis en faisant réagir le produit intermédiaire obtenu avec un aldéhyde de formule Z'''—CHO.

15. Procédé selon la revendication 13 pour la préparation d'azurants optiques asymétriques de formule:

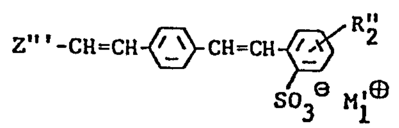

où Z''', $R_2''$ et $M_1'^{\oplus}$ sont définis comme dans la revendication 14, en faisant réagir un composé de phosphonium de formule:

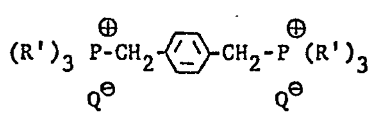

où R' et $Q^{\ominus}$ sont définis comme dans la revendication 14, avec un aldéhyde de formule:

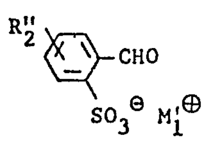

puis en faisant réagir le produit intermédiaire obtenu avec un aldéhyde de formule Z'''—CHO.

16. Procédé selon la revendication 10, caractérisé par le fait qu'on effectue le premier stade en présence d'un alcoolate de sodium ou de potassium dans un alcool aliphatique primaire, inférieur, comme solvent à des températures comprises entre 20° et 100°C, et qu'on effectue le deuxième stade, après séparation du produit intermédiaire, en présence d'un alcoolate de sodium ou de potassium, d'un hydroxyde de sodium ou de potassium, ou de sodium ou de potassium métalliques, dans un solvant bouillant au moins à 100°C, de préférence dans un alcool supérieur commé l'éthylèneglycol à des températures comprises entre 100° et 200°C.